# FASCICULE DE BREVET EUROPEEN

(11) **EP 2 342 566 B1**
(45) Date de publication et mention de la délivrance du brevet: **28.01.2015**
(21) Numéro de dépôt: 09748405.9
(22) Date de dépôt: 17.09.2009
(51) Int. Cl.: G01N 33/564

(54) **PROCÉDÉ DE DIAGNOSTIC D'UNE SCLÉRODERMIE SYSTÉMIQUE OU D'UNE HYPERTENSION ARTÉRIELLE PULMONAIRE**
VERFAHREN ZUR DIAGNOSE VON SKLERODERMIE ODER PULMONAL-ARTERIELLER HYPERTONIE
METHOD FOR THE DIAGNOSIS OF SYSTEMIC SCLERODERMA OR OF PULMONARY ARTERIAL HYPERTENSION

(30) Priorité: 17.09.2008 FR 0856255
(43) Date de publication de la demande: 13.07.2011
(73) Titulaire: Assistance Publique Hôpitaux De Paris, 75004 Paris (FR); Université Paris Descartes, 75006 Paris (FR); Université Paris-Sud 11, Paris (FR)
(72) Inventeur: MOUTHON, Luc, F-94160 Saint Mande (FR); HUMBERT, Marc, F-92130 Issy Les Moulineaux (FR); CALZAS, Cynthia, F-93370 Montfermeil (FR); CAMOIN, Luc, F-75013 Paris (FR); SAHBATOU, Younes, F-93200 Saint-Denis (FR)
(74) Mandataire: Sekhri, Redha
(86) Numéro de dépôt international: PCT/FR2009/051753
(87) Numéro de publication internationale: WO 2010/031968

(56) Documents cités:
- WO-A2-2009/055880
- US-A1- 2002 009 749
- PRATESI FEDERICO ET AL: "Autoantibodies specific for alpha-enolase in systemic autoimmune disorders" JOURNAL OF RHEUMATOLOGY, TORONTO, CA, vol. 27, no. 1, 1 janvier 2000 (2000-01-01), pages 109-115, XP009094488 ISSN: 0315-162X
- SAHBATOU Y ET AL: "Les anticorps anticellules musculaires lisses vasculaires sont presents dans le serum des patients ayant une sclerodermie systemique et/ou une hypertension arterielle pulmonaire" REVUE DE MEDECINE INTERNE, CMR, ASNIERES, FR, vol. 30, 1 juin 2009 (2009-06-01), page S62, XP026129318 ISSN: 0248-8663 [extrait le 2009-05-20] & Société Nationale Francaise de Médecine Interne: "Programme - 59ème congrès" juin 2009 (2009-06), XP002563728 Extrait de l'Internet: URL:http://www.snfmi.mcocongres.com/59/ima ges/programme.pdf> [extrait le 2010-01-18]
- GRASSEGGER ALFRED ET AL: "Autoantibodies in systemic sclerosis (scleroderma): clues for clinical evaluation, prognosis and pathogenesis." WIENER MEDIZINISCHE WOCHENSCHRIFT (1946) 2008, vol. 158, no. 1-2, janvier 2008 (2008-01), pages 19-28, XP002513815 ISSN: 0043-5341
- TERRIER BENJAMIN ET AL: "Identification of target antigens of antifibroblast antibodies in pulmonary arterial hypertension." AMERICAN JOURNAL OF RESPIRATORY AND CRITICAL CARE MEDICINE 15 MAY 2008, vol. 177, no. 10, 15 mai 2008 (2008-05-15) , pages 1128-1134, XP008101530 ISSN: 1535-4970
- DIB HANADI ET AL: "Identification of target antigens of endothelial cells antibodies in systemic sclerosis and idiopathic pulmonary arterial hypertension" ARTHRITIS AND RHEUMATISM, LIPPINCOTT, PHILADELPHIA, US, vol. 58, no. 9, 5 septembre 2008 (2008-09-05), page S837, XP008101595 ISSN: 0004-3591 [extrait le 2008-10-29]
- ULANET DANIELLE B ET AL: "Selective cleavage of nucleolar autoantigen B23 by granzyme B in differentiated vascular smooth muscle cells: insights into the association of specific autoantibodies with distinct disease phenotypes." ARTHRITIS AND RHEUMATISM JAN 2004, vol. 50, no. 1, janvier 2004 (2004-01), pages 233-241, XP002513816 ISSN: 0004-3591
- MOUTHON L ET AL: "Pulmonary arterial hypertension: an autoimmune disease?" THE EUROPEAN RESPIRATORY JOURNAL : OFFICIAL JOURNAL OF THE EUROPEAN SOCIETY FOR CLINICAL RESPIRATORY PHYSIOLOGY DEC 2005, vol. 26, no. 6, décembre 2005 (2005-12), pages 986-988, XP002513817 ISSN: 0903-1936 cité dans la demande
- FRITZLER ET AL: "Challenges to the use of autoantibodies as predictors of disease onset, diagnosis and outcomes", AUTOIMMUNITY REVIEWS, ELSEVIER, AMSTERDAM, NL, vol. 7, no. 8, 9 July 2008 (2008-07-09), pages 616-620, XP025408622, ISSN: 1568-9972, DOI: 10.1016/J.AUTREV.2008.06.007 [retrieved on 2008-07-09]
- TERRIER BENJAMIN ET AL: "Anti-fibroblast antibodies from systemic sclerosis patients bind to alpha-enolase and are associated with interstitial lung disease", ARTHRITIS & RHEUMATISM, JOHN WILEY & SONS, INC, US, vol. 58, no. 9, 29 October 2008 (2008-10-29), page S836, XP008101458, ISSN: 0004-3591 [retrieved on 2008-10-29]

## Description

L'invention concerne un procédé *in vitro* de détection d'une sclérodermie systémique (ScS) ou d'une hypertension artérielle pulmonaire associée à une sclérodermie (HTAP-ScS) ou d'une hypertension artérielle pulmonaire idiopathique (HTAPi) chez un sujet, comprenant la détermination de la présence et/ou de la quantité d'anticorps dans un échantillon biologique provenant d'un patient comme décrit dans les revendications.

### Etat de la technique

La sclérodermie systémique (ScS) est une maladie rare qui se caractérise par la survenue de lésions de fibrose intéressant la peau et certains viscères comme le poumon, le tube digestif et le coeur, ainsi qu'une hyperréactivité vasculaire à l'origine d'un phénomène de Raynaud et de manifestations sévères comme la crise rénale et l'hypertension artérielle pulmonaire (HTAP). La physiologie de la ScS est complexe et partiellement comprise. La survenue d'une ScS résulte du dysfonctionnement de trois types cellulaires, les lymphocytes B et T responsables d'une dérégulation immunitaire, les cellules endothéliales responsables d'anomalies vasculaires et les fibroblastes responsables de lésions de fibrose.

Quatre-vingt dix pour cent des patients sclérodermiques ont des anticorps antinucléaire (AAN) dans leur sérum. Certains auto-anticorps sont très spécifiques de la ScS et mutuellement exclusifs, comme les anticorps anti-topoisomérase I (anti-SCI-70) (ATA) (Tamby et al., 2007), plus fréquemment présents dans les formes diffuses de la maladie, les anticorps anti-centromère (ACA), associés en règle aux formes cutanées limitées (Moroi et al., 1980), ou les anticorps anti-ARN polymérase III associés aux formes cutanées diffuses et à la survenue d'une crise rénale (Bunn et al., 1998). Les AAN n'ont pas de rôle pathogène démontré au cours de la ScS mais leur détection constitue une aide au diagnostic précoce de la ScS. D'autres auto-anticorps non spécifiques de la ScS comme les anticorps anti-ribonucléoprotéine, anti-SSA et anti-SSB, les anticorps anti-cardiolipine ou le facteur rhumatoïde sont quelquefois trouvés au cours de la ScS. Des anticorps anti-cellules endothéliales (AECA) peuvent être détectés chez 28 à 54 % des patients sclérodermiques. Ces autoanticorps sont capables d'induire l'expression de molécules d'adhésion et de provoquer une apoptose des cellules endothéliales en présence de cellules tueuses naturelles (« natural killer ») (Bordron et al., 1998). Les cibles des AECA au cours de la ScS sont aujourd'hui mal connues et à ce jour il n'a pas été identifié d'antigène spécifique de la cellule endothéliale. En revanche, l'ADN topoisomérase 1 (Garcia de la Pena-Lefebvre etal., 2004) et la protéine centromérique B (Servettaz et al., 2006) ont été identifiées comme des cibles des AECA chez les malades sclérodermiques. Des anticorps anti-fibroblastes (AFA) ont été identifiés chez les patients sclérodermiques. Ces anticorps sont capables d'activer les fibroblastes, d'augmenter l'expression de molécules d'adhésion comme l'inter-cellular adhésion molecule-1 (ICAM) et pro-inflammatoires (augmentation des niveaux d'ARNm, d'IL-1α, d'IL-1β et d'IL-6) ainsi que la synthèse de collagène (Chizzolini et al., 2002). Il a récemment été mis en évidence que les AFA pouvaient se fixer sur la topoisomérase 1 adsorbée à la surface des fibroblastes (Henault et al., 2006). En ELISA (Enzyme-linked immunosorbent assay), des AFA ont été retrouvés chez 30 % des patients présentant une HTAP associée à une ScS (Tamby et al., 2006).

L'hypertension artérielle pulmonaire (HTAP) est une pathologie rare responsable de la survenue d'une décompensation cardiaque droite pouvant aboutir au décès. Le diagnostic d'HTAP est établi par un cathétérisme droit qui permet la mesure de la pression artérielle pulmonaire moyenne supérieure ou égale à 25mmHg au repos en l'absence d'élévation de la pression capillaire pulmonaire (Rubin, 1997). La survenue d'une HTAP est la conséquence d'une obstruction chronique des petites artères pulmonaires secondaire à la prolifération de cellules endothéliales, de cellules musculaires lisses vasculaires et de fibroblastes (Dorfmuller et al, 2003). En particulier, la néo-muscularisation des petites artères pulmonaires périphériques normalement non musculaires est une caractéristique commune à toutes les formes de remodelage associées à l'HTAP.

L'HTAP peut être idiopathique, c'est à dire sporadique, mais aussi familiale, associée à la prise d'anorexigènes (dexfenfluramine), ou associée à un certain nombre de pathologies comprenant l'infection par le virus de l'immunodéficience humaine (VIH). L'HTAP peut aussi être associée à des collagénoses comme la ScS (Hachulla et al, 2005), le syndrome de Sharp (ou connectivites mixte), ou plus rarement le lupus érythémateux systémique. Approximativement 8 à 12 % des patients sclérodermiques développent une HTAP, à l'origine d'une mortalité élevée. De plus, au cours de l'HTAP idiopathique, on trouve de temps à autres des stigmates d'auto-immunité, à savoir des anticorps anti-nucléaires ou des anticorps anti-thyroglobuline. La présence d'anticorps anti-cellules endothéliales (Tamby et al, 2005) et d'anticorps anti-fibroblastes (Tamby et al, 2006) a été rapportée au cours de l'HTAP idiopathique ou associée à la ScS. Cependant la valeur prédictive de ces anticorps dans la survenue de l'HTAP n'a pas été étudiée et le rôle potentiel des phénomènes autoimmuns dans la pathogénie de l'HTAP idiopathique reste incertain (Mouthon et al, 2005).

Dans la plupart des cas, l'HTAP est dépistée lorsque le patient présente une dyspnée de stade III ou IV. Lorsque le patient est suivi pour une maladie chronique comme la ScS, l'HTAP est dépistée par une échographie cardiaque annuelle.

La demande de brevet US 2002/0009749 décrit la détection d'anticorps anti-HMG-1 (high mobility group protein 1) et anti-HMG-2 pour le diagnostic de maladies auto-immunes, entre autres la sclérodermie systémique. Terrier et al. ont identifié les antigènes d'anticorps anti-fibroblastes présents chez des patients atteints d'hypertension artérielle pulmonaire (Terrier et al., 2008).

Un test simple et fiable de dépistage d'une ScS et/ou d'une HTAP fait toujours défaut, et serait précieux pour un diagnostic le plus précoce possible, qui permettrait de mettre en place rapidement des stratégies thérapeutiques pour améliorer l'état du patient et ses chances de survie. L'invention a pour objet un tel test.

### Résumé de l'invention

L'invention fournit maintenant un procédé in vitro de détection d'une sclérodermie systémique (ScS) ou d'une hypertension artérielle pulmonaire associée à une sclérodermie (HTAP-ScS) ou d'une hypertension artérielle pulmonaire idiopathique (HTAPi) chez un sujet, comprenant la détermination de la présence et/ou de la quantité d'au moins un anticorps choisi dans le groupe constitué des anticorps anti-Galectine-1, anti-protéine FAM10A4, anti-phosphoprotéine 1 induite par le stress, et anti-précurseur de la protéine de 78 kDa régulée par le glucose, dans un échantillon biologique provenant d'un patient,
la présence d'un anticorps anti-précurseur de la protéine de 78 kDa régulée par le glucose étant indicative d'une ScS ou d'une HTAPi ;
la présence d'un anticorps anti-protéine FAM10A4 étant indicative d'une HTAP-ScS ou d'une HTAPi ;
la présence d'un anticorps anti-galectine-1 étant indicative d'une ScS;
ou la présence en une quantité supérieure à une valeur contrôle d'un anticorps anti-phosphoprotéine 1 induite par le stress étant indicative d'une ScS, d'une HTAP-ScS ou d'une HTAPi.

### Description détaillée de l'invention

La sclérodermie systémique (ScS) et l'hypertension artérielle pulmonaire (HTAP) se caractérisent toutes deux par la présence d'autoanticorps (AAc) dans le sérum des patients, en particulier des AAc dirigés contre les cellules endothéliales et les fibroblastes. Avant les travaux présentés ci-dessous, aucun AAc dirigé contre les cellules musculaires lisses vasculaires (CMLV) n'avait été mis en évidence. La caractérisation par les inventeurs de tels anticorps dirigés contre les CMLV est d'une importance capitale, notamment eu égard au rôle clé de ces cellules dans la physiopathologie de la ScS, avec ou sans HTAP, et de l'HTAPi.

Les inventeurs se sont proposés d'identifier les anticorps dirigés contre les CMLV et d'en caractériser les cibles antigéniques. Pour ce faire, les inventeurs ont mis en oeuvre des CMLV d'artères mammaires internes comme source d'antigènes et testé des sérums de patients de phénotype identique (patients ayant une ScS sans ou avec HTAP, patients ayant une HTAP idiopathique, ou HTAPi, et sujets sains). Les anticorps ont été recherchés à l'aide d'une technique d'immunoblot en une puis deux dimensions suivie d'une identification des antigènes par spectrométrie de masse (voir la partie « exemples » ci-dessous).

Les inventeurs ont ainsi pu mettre en évidence des réactivités IgG nombreuses dont certaines très intenses avec l'ensemble des sérums de patients testés en immunoblot en une dimension, tandis que les sujets sains n'exprimaient quasiment aucune réactivité IgG. Les inventeurs ont caractérisé, en immunoblot en deux dimensions, plusieurs taches protéiques reconnues par au moins 80 % des IgG des pools de sérums d'un groupe de patients donnés, et non par les IgG sériques d'un pool de sujets sains et d'autres taches protéiques reconnues par la très grande majorité des IgG sériques des pools de malades avec une intensité plus forte que celle des IgG sériques du pool de sujets sains.

### Définitions :

Le terme « échantillon biologique » se réfère à tout échantillon biologique provenant d'un patient. Des exemples d'échantillons incluent des liquides biologiques et des biopsies tissulaires. Les fibroblastes de patients sclérodermiques cultivés à partir de biopsies de peau constituent également un exemple d'échantillon biologique. De manière préférentielle, l'échantillon peut être du sang, du sérum, de la salive, de l'urine, du sperme. De manière davantage préférée, l'échantillon biologique est un échantillon de sang ou de sérum.

Le terme « patient » se réfère à tout sujet susceptible d'être testé. De préférence il s'agit d'un humain, mais le terme inclut tout autre mammifère, tel que des chiens, chats, rongeurs, bétail, chevaux, singes etc. Le patient peut être testé quel que soit son sexe ou son âge. Le patient peut être un sujet à risque, être asymptomatique ou présenter des signes précoces ou avancés d'une ScS et/ou d'une HTAP. Par exemple le patient peut être un sujet prédisposé à développer une ScS et/ou une HTAP, en particulier un sujet portant une ou plusieurs mutations dans le gène codant pour BMPRII, endogline ou ALK1.

Le terme « diagnostic » signifie l'identification de la pathologie ou l'évaluation de l'état de sévérité de la pathologie.

Le terme « pronostic » signifie l'évaluation du risque d'aggravation, et de ses conséquences.

Le terme « valeur contrôle » se réfère à une valeur basale correspondant à la moyenne des valeurs obtenues avec l'échantillon biologique de sujets sains, non affectés par une ScS ou une HTAP ou une maladie susceptible d'entraîner une HTAP. Il peut s'agir d'une valeur statistique de référence.

Pour évaluer l'évolution de la pathologie, il peut être utile de tester un patient et de contrôler l'effet d'un traitement ou l'évolution de la pathologie, en testant de nouveau le patient, par exemple à plusieurs mois d'intervalle. Dans ce cas, les résultats du second test sont comparés aux résultats du premier test, ainsi que souvent à la valeur dite « contrôle ».

Une quantité d'anticorps « supérieure à la valeur contrôle » signifie généralement une augmentation statistiquement significative, par exemple d'au moins deux déviations standards au dessus de la moyenne des densités optiques des réactivités IgG de l'ensemble des sujets sains.

Par « antigène de capture », on entend un antigène, de préférence fixé sur une phase solide, qui est capable de retenir ledit au moins un anticorps présent dans un échantillon biologique, par liaison affine. L'antigène de capture peut être marqué.

Le terme « marqué » se réfère aussi bien à un marquage direct (par le biais d'enzymes, radioisotopes, fluorochromes, composés luminescents, etc...) qu'à un marquage indirect (par exemple, par le biais d'anticorps eux-mêmes marqués de manière directe ou à l'aide de réactifs d'une "paire d'affinité " marquée, telle que, mais non exclusivement, la paire avidine marquée-biotine, etc..).

### Anticorps identifiés :

Comme indiqué dans la partie « exemples », les inventeurs ont identifié plusieurs anticorps anti-CMLV chez des malades ayant une ScS avec ou sans HTAP, ou ayant une HTAPi.

La détection et/ou la quantification de ces anticorps peut être mise en oeuvre pour détecter une ScS et/ou une HTAP, pour réaliser le pronostic ou le suivi de ces pathologies, ou pour évaluer l'efficacité d'un traitement envers ces pathologies.

Les antigènes reconnus par les anticorps identifiés sont listés ci-dessous. Le nom et les numéros d'accès correspondant à ces antigènes dans la base de données de séquences protéiques SWISSPROT sont donnés dans les tableaux 1 et 2 de la partie « Exemples ».

Les inventeurs ont caractérisé plusieurs réactivités contre les CMLV dans les sérums de patients qui ne sont pas retrouvées dans les sérums de sujets sains. Les anticorps identifiés sont des anticorps anti-précurseur de la protéine de 78 kDa régulée par le glucose, anti-caldesmone, anti-protéine FAM10A4, anti-zyxine, anti-galectine 1, anti-précurseur A3 de la protéine disulfide-isomérase, anti-desmine, anti-périphérine, anti-ribonucléoprotéine H nucléaire hétérogène, anti-chaîne bêta de la tubuline et anti-polymerase I and transcript release factor. Dans un mode particulier de réalisation, le procédé décrit dans ce document comprend la détermination de la présence d'au moins un anticorps choisi dans le groupe constitué des anticorps anti-précurseur de la protéine de 78 kDa régulée par le glucose, anti-caldesmone, anti-protéine FAM10A4, anti-zyxine, anti-galectine 1, anti-précurseur A3 de la protéine disulfide-isomérase, anti-desmine, anti-périphérine, anti-ribonucléoprotéine H nucléaire hétérogène, anti-chaîne bêta de la tubuline et anti-polymerase I and transcript release factor dans un échantillon biologique provenant d'un patient, la présence dudit au moins un anticorps étant indicatrice d'une sclérodermie systémique et/ou d'une hypertension artérielle pulmonaire ou d'un risque de développer une sclérodermie systémique et/ou une hypertension artérielle pulmonaire.

Les inventeurs ont également caractérisé plusieurs réactivités qui ont une intensité significativement plus forte chez les patients que chez les sujets sains. Ces réactivités correspondent à des anticorps anti-vimentine, anti-phosphoprotéine 1 induite par le stress, anti-α-énolase, anti-triosphosphate isomérase, anti-précurseur de l'albumine sérique, anti-Isozyme L1 carboxyl-terminal hydrolase ubiquitine, anti-actine cytoplasmique 2, anti-péroxirédoxine-6, anti-Far-upstream element-binding protein 2 (ou anti-protéine 2) anti-précurseur de la réticulocabine-1, anti-γ-énolase, anti-précurseur mitochondrial de la péroxyde réductase dépendant de la thiorédoxine, anti-protéine d'activation de GTPase spécifique de Ran et anti-High mobility group protein B1. Dans un mode particulier de réalisation, les anticorps correspondant aux réactivités qui ont une intensité plus forte chez les patients que chez les sujets sains sont mis en oeuvre dans un procédé décrit dans ce document comprenant la comparaison de la quantité d'au moins un anticorps choisi dans le groupe constitué des anticorps anti-vimentine, anti-phosphoprotéine 1 induite par le stress, anti-α-énolase, anti-triosphosphate isomérase, anti-précurseur de l'albumine sérique, anti-Isozyme L1 carboxyl-terminal hydrolase ubiquitine, anti-actine cytoplasmique 2, anti-péroxirédoxine-6, anti-Far-upstream element-binding protein 2 (ou anti-protéine 2) anti-précurseur de la réticulocabine-1, anti-γ-énolase, anti-précurseur mitochondrial de la péroxyde réductase dépendant de la thiorédoxine, anti-protéine d'activation de GTPase spécifique de Ran et anti-High mobility group protein B1 dans un échantillon biologique provenant d'un patient à une valeur contrôle, la présence dudit au moins un anticorps en une quantité supérieure à la

valeur contrôle étant indicatrice d'une sclérodermie systémique et/ou d'une hypertension artérielle pulmonaire ou d'un risque de développer une sclérodermie systémique et/ou une hypertension artérielle pulmonaire.

L'invention concerne donc la mise en oeuvre d'au moins un anticorps dirigé contre les CMLV, dans un procédé, de préférence *in vitro,* de détection d'une ScS et/ou d'une HTAP.

De préférence, les anticorps dirigés contre les CMLV mis en oeuvre dans les procédés décrits sont choisis dans le groupe constitué des anticorps anti-précurseur de l'albumine sérique, anti-zyxine, anti-Galectine-1, anti-Isozyme L1 carboxyl-terminal hydrolase ubiquitine, anti-caldesmone, anti-protéine FAM10A4, anti-Far-upstream element-binding protein 2, anti-actine cytoplasmique 2, anti-γ-énolase, anti-précurseur A3 de la protéine disulfide-isomérase, anti-desmine, anti-périphérine, anti-ribonucléoprotéine H nucléaire hétérogène, anti-phosphoprotéine 1 induite par le stress, anti-triosphosphate isomérase, anti-péroxirédoxine-6, anti-réticulocabine-1, anti-péroxyrédoxine-2, anti-précurseur mitochondrial de la péroxyde réductase dépendant de la thiorédoxine, anti-protéine d'activation de GTPase spécifique de Ran, anti- High mobility group protein B1, anti-chaîne bêta de la tubuline et anti-polymerase I and transcript release factor.

Dans un mode particulier de réalisation, les anticorps dirigés contre les CLMV mis en oeuvre dans les procédés décrits sont choisis dans le groupe constitué des anticorps anti-précurseur de l'albumine sérique, anti-zyxine, anti-Galectine-1, anti-Isozyme L1 carboxyl-terminal hydrolase ubiquitine, anti-caldesmone, anti-protéine FAM10A4, anti-Far-upstream element-binding protein 2, anti-actine cytoplasmique 2, anti-γ-énolase, anti-précurseur A3 de la protéine disulfide-isomérase, anti-desmine, anti-périphérine, anti-ribonucléoprotéine H nucléaire hétérogène, anti-phosphoprotéine 1 induite par le stress, anti-triosphosphate isomérase, anti-péroxirédoxine-6 et anti-réticulocabine-1.

Les anticorps identifiés par les inventeurs peuvent être mis en oeuvre dans les procédés décrits seuls ou en combinaison. La détection et/ou la quantification peut être réalisée vis-à-vis d'un seul des anticorps identifiés, ou peut concerner une pluralité d'anticorps. On ainsi peut imaginer la réalisation du procédé sur un support solide, par exemple une microplaque, sur lequel sont disposés de manière définie et ordonnée les antigènes correspondant à la pluralité d'anticorps à détecter et/ou quantifier.

Selon un mode de réalisation de l'invention, les procédés décrits mettent en oeuvre la détection d'un anticorps anti-galectine 1 ou anti-phosphoprotéine 1 induite par le stress.

Selon un mode de réalisation de l'invention, les procédés décrits mettent en oeuvre la détection d'un anticorps anti-galectine 1 pour le diagnostic, le pronostic ou le suivi de la ScS. En effet, les inventeurs ont pu montrer que cet anticorps est spécifique de la ScS, eu égard à sa présence dans les sérums de patients ScS avec ou sans HTAP associée, et son absence dans les sérums de patients souffrant d'HTAPi.

Selon un autre mode de réalisation de l'invention, les procédés décrits mettent en oeuvre la détection d'un anticorps anti-précurseur de la protéine de 78 kDa régulée par le glucose pour le diagnostic, le pronostic ou le suivi de la ScS ou de l'HTAPi.

Selon un autre mode de réalisation de l'invention, les procédés décrits mettent en oeuvre la détection d'un anticorps anti-protéine FAM10A4 pour le diagnostic, le pronostic ou le suivi de l'HTAP, qu'elle soit idiopatyque ou associée à une ScS.

### Dosage des anticorps :

L'échantillon biologique est de préférence un échantillon de sérum, de préférence dilué au 1/100ème, ou plus, par exemple au 1/200ème ou 1/400ème.

De manière avantageuse, la quantité d'anticorps peut être déterminée par un immunoessai.

L'échantillon biologique peut être éventuellement traité dans une étape préalable, ou mis directement en présence d'au moins un antigène de capture.

Le procédé selon l'invention peut être réalisé selon divers formats bien connus de l'homme du métier: en phase solide ou en phase homogène; en un temps ou en deux temps; en méthode compétitive, à titre d'exemples non limitatifs.

Selon un mode de réalisation préféré, l'antigène de capture est immobilisé sur une phase solide. On peut utiliser, à titre d'exemples non limitatifs de phase solide, des microplaques, en particulier des microplaques de polystyrène, telles que celles commercialisées par la société Nunc, Danemark. On peut également utiliser des particules ou des billes solides, des billes paramagnétiques, telles que celles fournies par Dynal ou Merck-Eurolab (France) (sous la marque EstaporTM), ou encore des tubes à essai en polystyrène ou polypropylène, etc.

Un format d'immunoessai de détection des anticorps par compétition est également possible. D'autres modalités d'immunoessai sont encore envisageables et bien connues de l'homme du métier.

Dosages ELISA, radioimmunoessais, ou toute autre technique de détection peuvent être mis en oeuvre pour révéler la présence des complexes antigènes-anticorps formés.

Selon un mode particulier de réalisation préféré, l'antigène de capture correspond à une protéine entière ou à un fragment de ladite protéine. Par exemple, le procédé de l'invention comprend la mise en contact d'un échantillon biologique avec une protéine entière reconnue par l'anticorps à détecter et/ou quantifier. A titre illustratif, l'invention comprend la mise en contact d'un échantillon de sang ou de sérum avec la galectine 1 ou la phosphoprotéine 1 induite par le stress entière, pour la détection et/ou la quantification d'anticorps anti-galectine ou anti-phosphoprotéine 1 induite par le stress dans ledit échantillon.

Dans un exemple particulier, l'antigène de capture peut être couplé à une glutathion S transférase (GST), avant d'être déposé sur une microplaque.

A titre illustratif, les échantillons de sérum à tester, par exemple dilués au 1/100ème, sont mis à incuber sur la microplaque. Après lavage, des anticorps anti-Fcγ humain marqués (par exemple avec une phosphatase alcaline) sont ajoutés, les complexes étant révélés (par exemple par ajout d'un substrat de la phosphatase dont le clivage peut être détecté par lecture de l'absorbance).

### Patients visés :

Les patients visés sont ceux susceptibles de développer une ScS et/ou une HTAP.
Il peut s'agir d'un patient qui souffre d'une HTAP associée à une connectivite, telle qu'une sclérodermie systémique, au syndrome de Sharp (qui est une connectivite mixte), ou à un lupus érythémateux systémique.
Le patient peut également souffrir d'une HTAP idiopathique ou familiale.
Plus généralement, tout patient atteint d'une maladie vasculaire pulmonaire peut être avantageusement soumis au procédé de détection d'une HTAP tel que défini dans l'invention.
Par ailleurs, l'HTAP détectée peut être aussi une hypertension porto-pulmonaire (c'est-à-dire une HTAP associée à une hypertension portale), ou être associée à une cardiopathie congénitale, ou à une infection par le virus de l'immunodéficience humaine (VIH), ou encore être une hypertension pulmonaire post-embolique, compliquant l'évolution d'une bronchite chronique obstructive ou d'une cardiopathie cyanogène.
D'autres patients visés sont ceux exposés à certains médicaments coupe-faim comme la fenfluramine dont la prescription peut contribuer à la survenue d'une HTAP.
D'autres personnes susceptibles de bénéficier de ce type de test sont celles porteuses d'une mutation dans le gène codant pour BMPRII, endogline ou ALK1, et qui éventuellement ne présentent pas d'HTAP détectable à l'échographie, de façon à dépister les individus susceptibles de développer ultérieurement une HTAP.

### Evaluation de l'etficacité d'un traitement :

Un autre objet de ce document est un procédé *in vitro* d'évaluation de l'efficacité d'un traitement envers une ScS et/ou une HTAP, comprenant la détermination de la présence et/ou de la quantité d'au moins un anticorps tel que défini ci-dessus dans un échantillon biologique provenant d'un patient, à différents temps avant, au cours ou après le traitement, la diminution de la quantité dudit au moins un anticorps au cours du temps étant indicative d'une amélioration de la ScS ou de l'HTAP.
Le traitement classique actuel de l'HTAP associe un traitement symptomatique et un traitement vasodilatateur. Le traitement symptomatique associe des anti-coagulants, une oxygénothérapie et des diurétiques. Le traitement vasodilatateur repose sur les molécules suivantes : bloqueurs de canaux calcium, époprosténol (prostacycline) prescrit par voie intraveineuse en perfusion continue, les inhibiteurs des récepteurs de l'endothéline, sélectifs ou non, en particulier le bosentan, le sytaxentan et l'ambrysentan, les inhibiteurs des phosphodiestérases de type 5 en particulier le sildénafil, le taladafil, l'ensemble de ces médicaments étant administrés par voie orale, et l'iloprost inhalé, analogue de la prostacycline administré par voie inhalée. Ces traitements peuvent être éventuellement combinés. En cas d'échec de ces thérapeutiques, une transplantation pulmonaire ou coeur-poumons peut être proposée. Au cours de la ScS il est classique de prescrire des vasodilatateurs, en premier lieu des inhibiteurs calciques en traitement du phénomène de Raynaud, des inhibiteurs de la pompe à protons et un prokinétique, la domperidone, en traitement du reflux gastro-oesophagien. Les autres traitements dépendent des atteintes présentées par le patient : colchicine ou corticoides à faible dose en cas d'atteinte articulaire inflammatoire, inhibiteurs de l'enzyme de conversion en cas de crise rénale, cyclophosphamide en cas de pneumopathie infiltrante diffuse évolutive, traitement vasodilatateur d'une hypertension artérielle pulmonaire.

Les figures et exemples suivants illustrent l'invention sans en limiter la portée.

### Légendes des figures

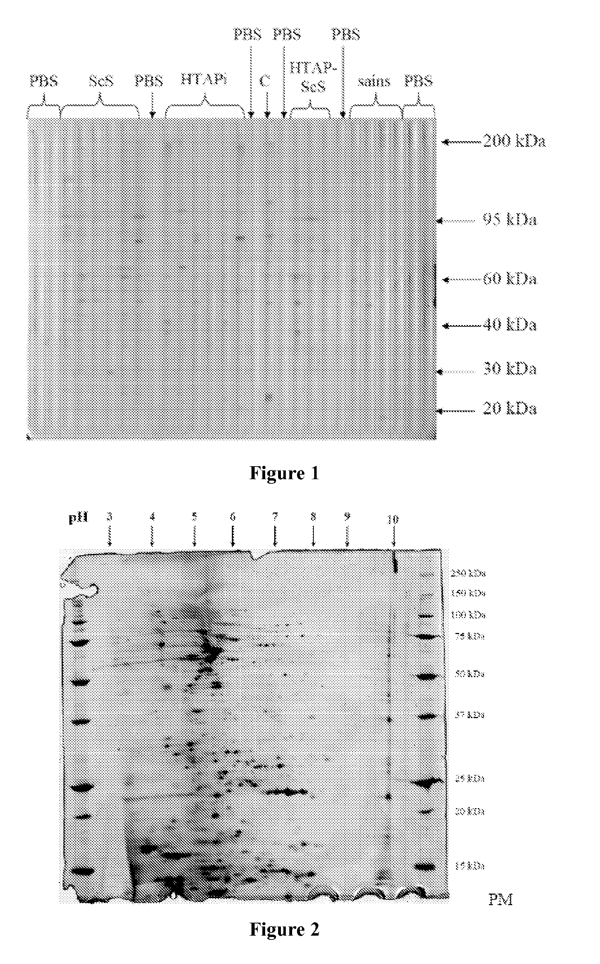

La **figure 1** correspond à un immunoblot à une dimension montrant les réactivités des IgG sériques de patients sclérodermiques avec (n=3) ou sans (n=6) HTAP, de patients ayant une HTAP idiopathique (n=6) et de sujets sains (n=4) vis-à-vis des protéines de cellules musculaires lisses vasculaires. L'HTAP était documentée par cathétérisme droit chez l'ensemble des patients. ScS : sclérodermie systémique ; HTAP : hypertension artérielle pulmonaire ; HTAPi : hypertension artérielle pulmonaire idiopathique ; HTAP-ScS : hypertension artérielle pulmonaire associée à une sclérodermie ; C : contrôle interne (HTAP-ScS) ; PBS : Phosphate Buffer Saline.
La **figure 2** correspond à un gel de référence en deux dimensions d'un extrait protéique total de cellules musculaires lisses vasculaires, coloré au nitrate d'argent. Première dimension (axe horizontal): pH 3-10, seconde dimension (axe vertical): gradient d'acrylamide 7-18 %, permettant le dénombrement de 880 taches protéiques.

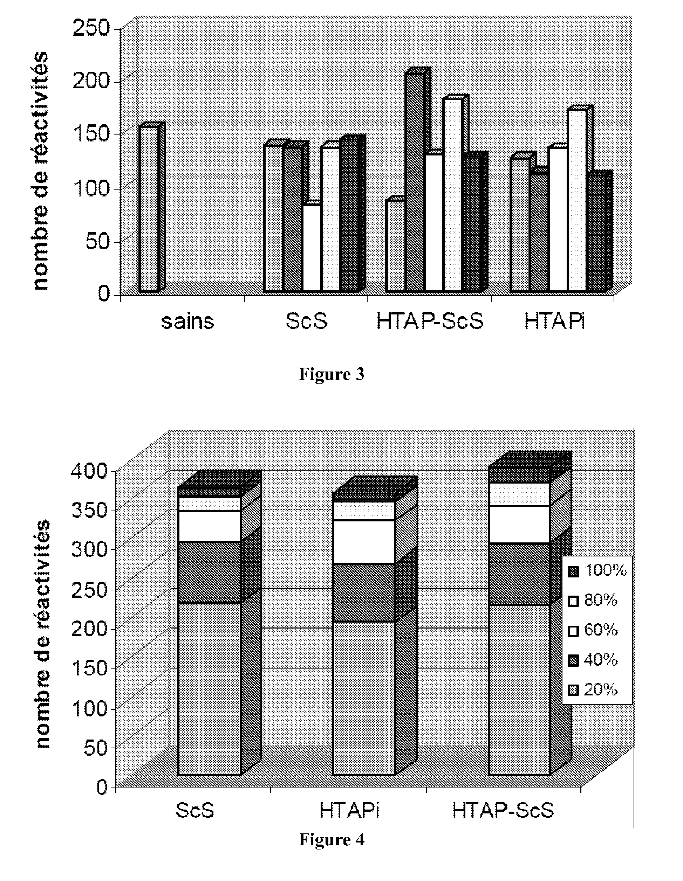

La **figure 3** est un graphique montrant le nombre de taches protéiques reconnues par les IgG de 15 pools de 3 sérums de patients phénotypiquement identiques ayant une sclérodermie systémique et/ou une HTAP et par un pool de 12 sujets sains après ajustement sur le gel de référence. L'échelle des ordonnées indique le nombre de réactivités IgG.
La **figure 4** montre la proportion des taches de réactivité reconnues par les IgG des sérums des pools de 3 patients au sein de chaque groupe (reconnues ou non par les sujets sains). 20 %, 40 %, 60%, 80%, 100% : nombre de taches protéiques reconnues respectivement par 1/5, 2/5, 3/5, 4/5, 5/5 des pools de patients dans un groupe donné.

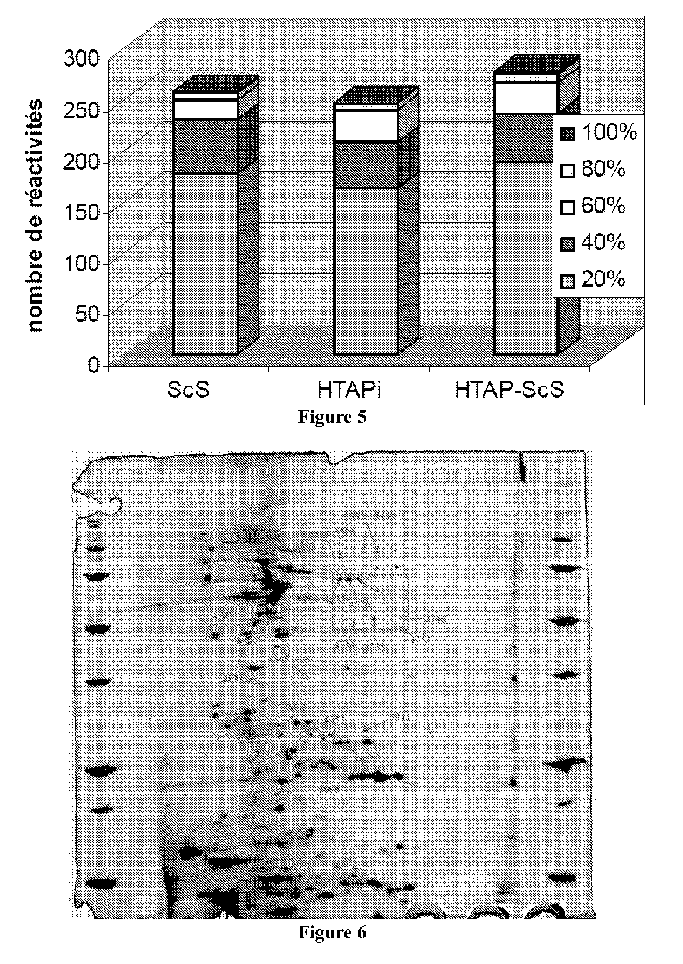

La **figure 5** représente le nombre de taches reconnues par les IgG des sérums des pools de patients de chaque groupe et non reconnus par les sérums des sujets sains. 20 %, 40 %, 60%, 80%, 100% : nombre de taches protéiques reconnues respectivement par 1/5, 2/5, 3/5, 4/5, 5/5 des pools de patients dans un groupe donné.
La **figure 6** montre la localisation des taches protéiques candidates sur le gel d'électrophorèse en deux dimensions d'un extrait protéique total de cellules musculaires lisses vasculaires, coloré au nitrate d'argent. Première dimension (axe horizontal): pH 3-10, seconde dimension (axe vertical): gradient d'acrylamide 7-18 %.

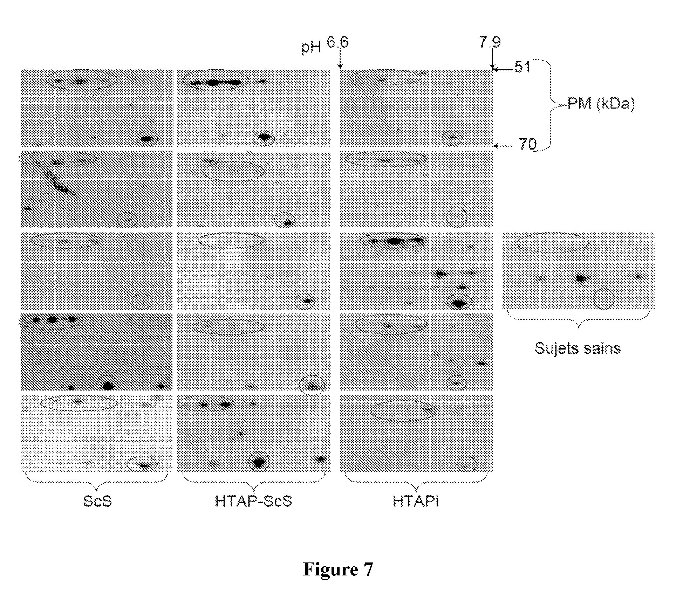

La **figure 7** montre l'intensité des réactivités des IgG des 15 pools de 3 sérums de patients et du pool de sérums de sujets sains dirigées contre l'α-énolase et la phosphoprotéine 1 induite par le stress sur des membranes de PVDF des différents groupes de malades. La zone de membrane de PVDF figurée pour chacun des groupes correspond à un pHi compris entre 6.6 et 7.9 et des PM compris entre 51 et 70 kDa.

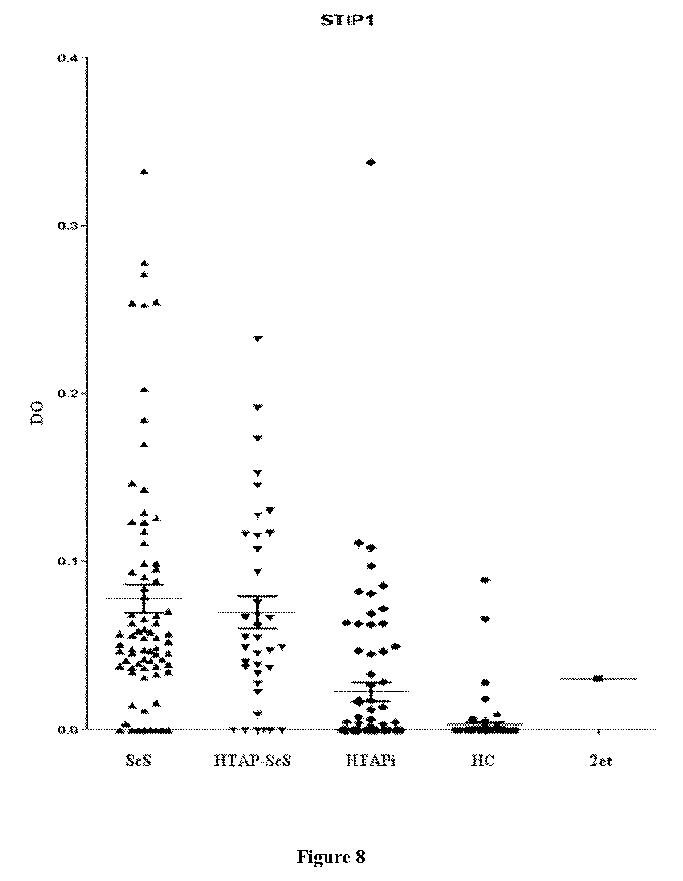

La **figure 8** est une représentation graphique de la détection d'anticorps anti-phosphoprotéine 1 induite par le stress (stress induced phosphoprotein 1 ou STIP1) par ELISA dans les sérums de patients atteints d'une ScS, d'une HTAPi, d'une HTAP associée à une ScS et dans les sérums de contrôles sujets sains (HC). Les données rapportées correspondent à la densité optique des protéines à 405 nm (DO405), le bruit de fond (DO405 du tampon bicarbonate) ayant été soustrait. Chaque point représente la réactivité d'un échantillon de sérum. Les barres horizontales uniques indiquent la moyenne et les doubles barres horizontales indiquent l'écart type. Les échantillons sont considérés positifs lorsque la densité optique est supérieure ou égale à la moyenne + 2 écarts-types du contrôle (2et).

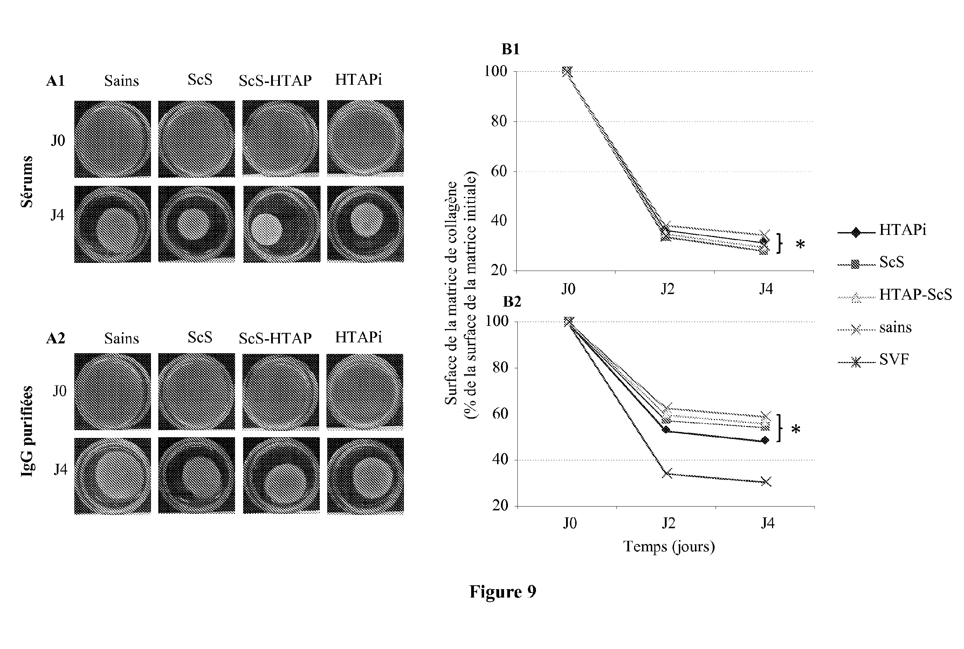

La **figure 9** montre l'effet du sérum et des IgG purifiées de patients ayant une ScS, une ScS-HTAP ou une HTAPi sur la contraction d'une matrice de collagène par les CMLV d'aorte. La contraction de matrices de collagène ensemencées avec des CMLV a été suivie pendant 4 jours, en présence de SVF, de sérum, ou d'IgG purifiées de patients ayant une ScS, ScS-HTAP, ou une HTAPi. A, photographies de 4 matrices correspondant aux 4 conditions, incubées avec le sérum (A1) ou les IgG purifiées (A2), à J0 et J4. B, représentation graphique de la cinétique de contraction de matrices de collagène incubées avec le sérum (A1) ou les IgG purifiées (A2) de patients ScS, ScS-HTAP ou HTAPi, ou de sujets sains appariés. Pour chaque conditions, 10 sérums ou IgG purifiées ont été utilisés. *Sérums: Sains/ScS p = 0,012; IgG purifiées Sains/HTAPi p = 0,001.

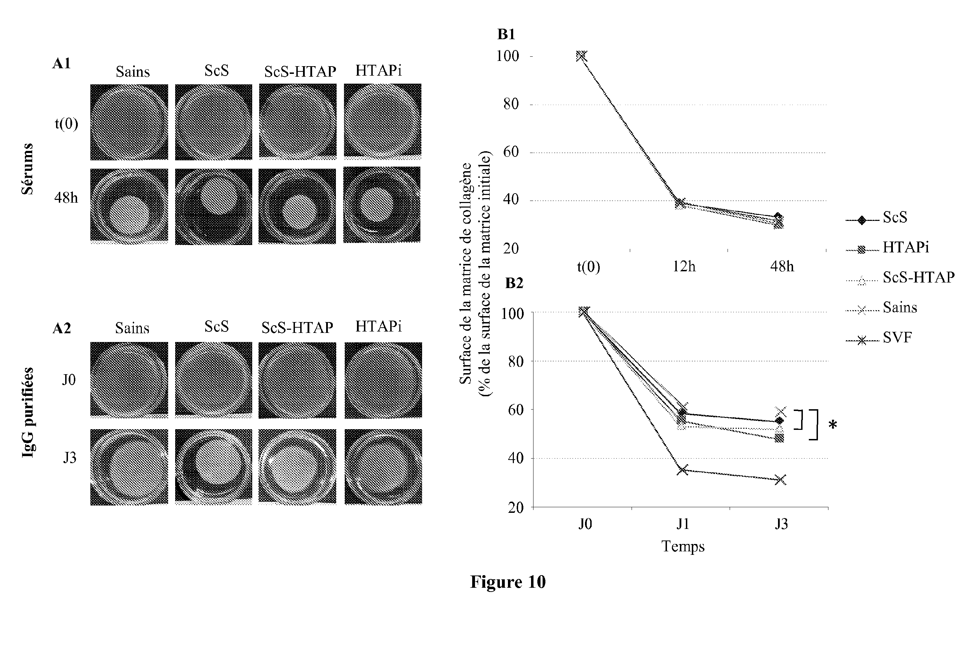

La **figure 10** montre l'effet du sérum et des IgG purifiées de patients ayant une ScS, une ScS-HTAP, ou une HTAPi sur la contraction d'une matrice de collagène par des CMLV activées par du TNF-α. La contraction de matrices de collagène ensemencées avec des CMLV a été suivie pendant deux (sérums) ou trois jours (IgG purifiées), en présence de SVF, de sérum, ou d'IgG purifiées de patients ayant une ScS, une ScS-HTAP, ou une HTAPi. A: photographies de 4 matrices correspondant aux 4 conditions, incubées avec le sérum (A1) ou les IgG purifiées (A2), à J0 et J2 (sérum) ou J3 (IgG purifiées). B: représentation graphique de la cinétique de contraction de matrices de collagène incubées avec le sérum (B1) ou les IgG purifiées (B2) de patients ScS, ScS-HTAP ou HTAPi, ou de sujets sains appariés. Pour chaque condition, 10 sérums ou IgG purifiées ont été utilisés. *Sains/HTAPi p = 0,001; Sains/ScS-HTAP p = 0,029

### Exemple 1

### Matériels et méthodes

### Sérums

Les inventeurs ont utilisé les sérums de patients ayant une HTAPi ou une ScS avec ou sans HTAP. L'HTAP était dépistée par une échographie cardiaque trans-thoracique et confirmée par un cathétérisme droit. Les patients sclérodermiques répondaient aux critères de l'American Rheumathology Association (ARA) et/ou aux critères de Leroy et Medsger. Les sérums étaient recueillis et conservés à -80°C avant leur utilisation. Tous les patients avaient signé un consentement éclairé dans le cadre de l'étude HTAP-Ig (Contrat d'Investigation et de Recherche Clinique 2005 N° CRC 05066, promoteur Assistance Publique-Hôpitaux de Paris).

Dans un premier temps les sérums de 15 patients ayant une HTAPi, 15 patients ayant une HTAP-ScS, 15 patients ayant une ScS sans HTAP et 12 sujets sains ont été testés dans des expériences d'immunoblot 1 D. Dans un deuxième temps, les mêmes sérums ont été testés sous forme de pools de 3 sérums de patients ayant un phénotype similaire. Un pool de 12 sérums de sujets sains, différents de ceux utilisés en 1 D a été utilisé comme témoin dans les expériences d'immunoblot en 2 D.

### Cellules

Des CMLV humaines obtenues à partir d'artères mammaires chez des patients ayant subi un pontage aorto-coronaire nous ont été fournies par le Dr Babett Weksler (Institut Cochin, Paris). Ces cellules ont été immortalisées après transduction séquentielle lentivirale de la sous-unité catalytique de l'holoenzyme humaine Telomerase Reverse Transcriptase et de l'antigène T du polyomavirus SV40 (Simian Virus 40) dans une culture primaire de CMLV adultes (Weksler et al., 2005). Ces cellules ont été cultivées sur des flasques de 175 cm² dans du milieu de culture Smooth Muscle Cell Growth Médium 2 (PromoCell, Heidelberg, Allemagne) supplémenté avec 5 % de sérum de veau foetal décomplémenté (SVF), 0,5 ng/ml d'Epithelial Growth Factor (EGF), 2 ng/ml de basic Fibroblast Growth Factor, 5 µg/ml d'insuline, 1% de pénicilline/streptomycine et 1 % de ciprofloxacine. Elles ont été utilisées pour les expériences d'immunoblot en 1 D et 2D.
Des CMLV d'aorte humaine (Cambrex) ont été utilisées dans les expériences évaluant l'effet du sérum et des IgG purifiées de patients ayant une ScS, une ScS-HTAP, ou une HTAPi sur la contraction d'une matrice de collagène par des CMLV non activées ou activées par du TNF-α

### Extraction des protéines

### Electrophorèse en une dimension

Les CMLV arrivées à confluence étaient décollées à la trypsine, lavées avec du phosphate buffer saline (PBS) puis centrifugées à 1600 tr/min à 20°C. Le culot cellulaire était ensuite récupéré dans un tampon contenant 2 % de sodium dodecyl sulfate (SDS), Tris 62.5 mM pH 6.8, β-mercaptoéthanol 5 % en présence d'inhibiteurs de protéases : 1 µg/ml de pepstatine, d'aprotinine, de leupeptine et 1 mM de phenylmethylsulphonyl fluoride (PMSF). Le mélange était ensuite soniqué à 4 reprises 30 sec dans la glace à 4 °C à une puissance de 25 W puis chauffé 10 min à 100 °C. Les extraits protéiques étaient ensuite aliquotés et conservés à -80 °C jusqu'à utilisation.

### Electrophorèse en deux dimensions

Les CMLV arrivées à confluence étaient lavées 2 fois dans du PBS dépourvu de Mg²⁺ et de Ca²⁺, puis décollées et récupérées dans une solution isotonique en l'absence d'enzyme contenant des agents chélateurs comme l'EDTA (Cell Dissociation Buffer enzyme free PBS-based, Invitrogen, Carlsbad, CA, Etats-Unis (EU)). Les cellules étaient récoltées puis centrifugées 5 min à 1300 rpm à 20 °C. Après un lavage dans une solution de NaCl isotonique, une deuxième centrifugation était réalisée. Après avoir répété cette opération une deuxième fois, le culot cellulaire était congelé à -80 °C en présence d'1 mM de PMSF et d'un cocktail d'inhibiteur de protéases (Complete Mini, Roche Diagnostic, Meylan, France).

Dans un second temps, les protéines étaient extraites après trois sonications de 30 s chacune à 4°C dans un tampon composé d'urée 5M, de thiourée 2M, de 3-[(3-cholamidopropyl) dimethylammonio]-1-propane sulfonate (CHAPS) 2 %, de Tris 40mM et d'ampholytes Bio-Lyte 3/10 0.2 % (ReadyPrep Sequential Extraction Reagent 3, Bio-Rad, Hercules, CA, EU). Deux ultracentrifugations à 150 000 g pendant 25 min chacune étaient ensuite réalisées à 4°C (Optima LE-80K, Beckman, Fullerton, CA, EU). Afin d'éviter la perturbation artéfactuelle de l'ADN libéré au cours de la sonication, une congélation à -80°C était réalisée afin de faire précipiter l'ADN. L'extrait était ensuite décongelé, centrifugé et le surnageant récupéré. Enfin, la concentration en protéines était mesurée par la méthode de Lowry (RC DC Protein Assay, Bio-Rad, Richmond, EU). Du dithiothreitol (DTT) était ajouté à l'extrait à une concentration finale de 64mM avant congélation à -80°C.

### Immunoblot 1D

### Séparation des protéines par électrophorèse monodimensionnelle

Les protéines de l'échantillon étaient séparées selon leur poids moléculaire (PM) sur des gels de polyacrylamide dénaturants en présence de SDS (SDS-PAGE) à 10 % d'acrylamide (10 % acrylamide, 0.27 % bis acrylamide, 0.375 M Tris/HCl pH 8.8, 0.1 % SDS, 0.1 % Ammonium Persulfate, 0.04 % de Tetramethylethylenediamine (TEMED) (Biorad, Hercules, CA, EU)). Cent vingt microlitres de protéines étaient déposés en haut de chaque gel et la migration s'effectuait dans un tampon de migration (25 mM Tris/HCl, 192 mM glycine, 0.1 % SDS) à 25 mA par gel à ampérage constant avec un dispositif mini-PROTEAN III (Bio Rad) pendant environ 50 min.

### Electrotransfert à partir de gels en une dimension

Les protéines ainsi séparées étaient transférées du gel sur une membrane de nitrocellulose (Immunetics Inc., Boston, Massachusetts, EU) grâce à un module d'électrotransfert semi-sec (Semi Dry Electroblotter A ANCOS, Hoejby, Danemark) pendant 1 h à 50 mA par module de transfert. Les membranes étaient ensuite bloquées 1h30 dans du PBS Tween 20 0.2 % (Sigma) et incubées toute la nuit en présence de sérums appartenant à l'un des trois groupes suivant : ScS associée ou non à une HTAP, HTAPi. Les sérums de 12 sujets sains étaient utilisés comme contrôles et du PBS-Tween 0.2 % seul sans Ac avait été utilisé comme témoin négatif. Chaque sérum de patients était dilué au ½ dans du PBS-Tween 0.2 % et les sérums de sujets sains étaient dilués au 1/100.

Après 5 lavages courts de 20 s et 5 lavages longs de 5 min dans une solution de PBS-Tween 0.2 %, les membranes étaient incubées pendant 1h30 à 20 °C avec un Ac secondaire anti-IgG humaines spécifique du fragment Fcγ humain (Ac anti-Fcγ humain) conjugué à la phosphatase alcaline (Dako, Glostrup, Danemark). Après 5 lavages courts et 1 lavage long dans du PBS-Tween 0.2 %, les membranes ont été lavées dans une solution de tampon de Tris Buffer Saline (TBS : Tris 24mM, NaCl 136,9mM, KCI 18,6mM, pH 8) et les réactivités étaient révélées à l'aide du substrat de la phosphatase alcaline (bromo-chloro-indolyl phosphate et nitroblue tetrazolium (Sigma)) dans un tampon contenant du Tris 100 mM, NaCl 100 mM, MgCl2 5 mM (VWR international). La réaction était stoppée par des lavages à l'eau bi-distillée, et les membranes étaient séchées puis scannées à l'aide d'un scanner de haute résolution (Perfection 1200S, Seiko Epson Corporation, Hirooka, Japon).

### Immunoblot 2D

### Isoélectrofocalisation (IEF)

L'IEF permet la séparation des protéines selon leur pH isoélectrique (pHi). Cette étape était réalisée en gradient de pH immobilisé (IPG), c'est-à-dire qu'elle s'était opérée sur un gel d'acrylamide coulé sur une bandelette rigide (strip) dans lequel un gradient de pH avait été préformé, ici, un gradient de 3 à 10 (ReadyStrip 17cm, pH 3-10, Bio-Rad). Les bandelettes étaient placées dans une cuve horizontale Bio-Rad de type Protean IEF cell à température ambiante. Chaque strip était placé dans une rainure en présence d'un mélange contenant du tampon de réhydratation et 100µg d'extraits protéiques de CMLV; le tout était recouvert de 2 ml d'huile minérale afin de limiter l'évaporation. Le tampon de réhydratation était constitué d'urée ultra-pure 7M (VWR, Fontenay-Sous-Bois, France), thiourée 2M (Sigma), CHAPS 4 % (Sigma), triton X100 0.002 % (Sigma), DTT (Sigma), bleu de bromophénol, et ampholytes Pharmalytes 3-10 (Amersham Biosciences, Uppsala, Suède).

L'IEF comprenait une hydratation passive de 9 h suivie d'une hydratation active de 12 h des strips sous une tension de 50 V. Ensuite, l'IEF était réalisée comme suit : 1 h à 200 V (élimination des excès de sels), puis montée linéaire de la tension pendant 1 h jusqu'à 1000 V, puis pendant 6 h jusqu'à 10 000 V, puis pendant 1 h jusqu'à 10 000 V.

### Séparation des protéines en gel d'acrylamide (SDS-PAGE)

Cette deuxième étape permettait la séparation des protéines selon leur PM. Douze gels de 20x20x0,1 cm avec un gradient d'acrylamide de 7 à 18,5 % étaient coulés simultanément dans une cuve multi-gels (Protean Plus Multi-Casting Chamber, Bio-Rad) assurant une reproductibilité optimale et permettant la séparation de protéines ayant un PM compris entre 10 et 250 kDa. Avant de réaliser la deuxième dimension, les strips obtenus à la fin de l'étape précédente étaient mis en présence de 2 tampons d'équilibration afin de réduire et alcaliniser les groupements sulfhydryl des cystéines. Le premier tampon d'équilibration se composait de Tris 50 mM, urée 6 mM, glycérol 40 %, SDS 52 mM et DTT 32,4 mM. Le second tampon était composé de Tris 50mM, urée 6mM, glycérol 40 %, SDS 52 mM et iodoacétamide 86,5 mM. Ensuite, les strips étaient maintenus au contact des gels d'acrylamide dans une solution d'agarose 1 % (Agarose Low Melting Point Ultrapure, Gibco BRL Invitrogen) contenant du bleu de bromophénol pour suivre le front de migration. Des marqueurs de PM avaient été disposés de part et d'autre du strip. La migration durait environ 30 h dans un tampon de migration (Tris 25 mM, glycine 192 mM, SDS 3,5 mM, Thiosulfate de sodium 1,25 mM (Sigma)) maintenue à 10°C (Cuve Protean Plus Dodeca Cell Bio-Rad, Cryostat MultiTemp III Amersham Biosciences) à ampérage constant; 40 V pendant 1 h puis 80 V pendant 1 h et enfin 15mA/gel jusqu'à ce que le front de migration soit sorti des gels.

A la fin de la migration dans la deuxième dimension, 11 gels étaient transférés sur des membranes de PolyVinyliDene Fluoride (PVDF) (Immobilon-P Transfer Membranes, pores de 0,45µm, Millipore, Bedford, MA, EU), tandis que le dernier gel était coloré au nitrate d'argent.

### Electro-transfert

Le transfert semi-sec s'effectuait à 4°C pendant 1h30 à ampérage constant (320mA). A la fin du transfert, les membranes étaient plongées 5 min dans une solution de PBS composé de NaCl 148 mM, de NaH2PO₄,2H2O 3,5 mM, de Na2HPO₄, 12H2O 17,6 mM, puis séchées.

### Coloration des gels non transférés

Le gel non transféré (appelé aussi gel de référence) était coloré au nitrate d'argent (Rabilloud et al., 1990) en 5 étapes; fixation (30 % éthanol absolu, 5 % acide acétique), lavage (nitrate d'argent 11,8 mM (Sigma), formaldéhyde 3,45mM (Sigma), coloration (nitrate d'argent 0.02 %) et enfin révélation (formaldéhyde 37 %, carbonate de sodium, thiosulfate). Le gel était ensuite conservé dans une solution de préservation (dimethylsulfoxide 2 % (Sigma), acide acétique 10 % avant d'être scanné à l'aide d'un densitomètre (GS-800, Bio-Rad).

### Incubation des membranes de PVDF avec les sérums et révélation des réactivités

Les membranes de PVDF initialement bloquées avec du PBS-Tween 0.2 % étaient incubées toute la nuit en présence de sérums de patients appartenant à l'un des trois groupes décrits précédemment: HTAPi, HTAP-ScS ou ScS sans HTAP. Pour chaque membrane, un pool de trois sérums appartenant à un même groupe dilué au 1/100ème dans une solution de PBS-Tween 0.2 % était utilisé. Pour chaque expérience, une membrane était incubée avec un pool de 12 sérums de sujets sains dilué dans le même tampon au 1/100ème. La révélation des réactivités s'était effectuée comme précédemment dans le cas de l'immunoblot 1D (Ac anti-Fcγ humain conjugué à la phosphatase alcaline, révélation des réactivités à l'aide du substrat de la phosphatase alcaline) puis les membranes étaient séchées et photographiées à l'aide d'un densitomètre (GS-800, Bio-Rad). Les membranes étaient ensuite colorées à l'or colloïdal (Protogold®, BioCell, Cardiff, GB) afin de révéler l'ensemble des protéines transférées à la surface des membranes. Une nouvelle acquisition densitométrique était ensuite effectuée.

### Analyse informatique

L'analyse informatique des gels et membranes était réalisée à l'aide d'un logiciel conçu spécialement pour l'analyse des gels bidimensionnels (Image Master 2D® Platinum 6.0, Buckinghamshire, Angleterre). La première étape consistait en une détection automatique des taches protéiques en fonction des paramètres que nous avions choisi (nombre de lissages effectués pour éliminer le bruit de fond, seuil Laplacien et surface minimale des taches à détecter). Les taches détectées étaient contrôlées visuellement à l'aide de procédés de reconstruction en 3 dimensions, de façon à éliminer les faux positifs et voir les taches de réactivité non détectées par le logiciel. Ensuite, chaque tache protéique reconnue par les IgG d'un individu était appariée avec la protéine correspondante à l'aide de la photographie densitométrique de la même membrane réalisée après coloration à l'or colloïdal. Cette étape était réalisée pour chacune des 16 membranes. Enfin, les protéines transférées sur les membranes étaient appariées aux protéines du gel choisi comme gel de référence. Cela permettait de collecter toutes les informations sur le gel de référence et de pouvoir ensuite comparer les taches protéiques reconnues par les sujets sains et les patients au sein des différents groupes étudiés.

### Spectrométrie de masse

Les taches protéiques reconnues comme cibles antigéniques était extraites par carottage d'un nouveau gel d'acrylamide chargé avec 400 µg d'extraits protéiques et coloré au bleu de Coomassie. Chaque spot carotté était placé dans le puit d'une plaque de 96 puits et digéré en présence de trypsine (Promega, France) pendant toute une nuit. Les échantillons digérés étaient ensuite transférés sur une autre plaque de 96 puits entreposée par la suite à 4° C avant l'analyse par spectrométrie de masse type 'Matrix-Assisted Laser Desorption Ionization-Time-of-Flight' (MALDI-TOF) (PerSeptive Biosystems Framingham, MA, EU.

### Dosage d'anticorps anti-STIP1 par ELISA

La phosphoprotéine 1 induite par le stress (STIP1) a été obtenue auprès de la société Tebu-bio (Tebu-bio, Maryland, USA), diluée dans un tampon de bicarbonate et déposée sur des plaques 96 puits (Maxisorb, NalgeNunc Int. Rochester, NY, USA) à une concentration finale de 3µg/mL à 4°C. La réactivité des IgG sériques obtenues à partir de 75 patients sclérodermiques sans HTAP, 74 atteints d'HTAPi, 37 patients sclérodermiques avec HTAP (ScS-HTAP), et 70 sujets sains (HC) ont été testés par ELISA contre STIP-1. Les puits ont été lavés cinq fois avec du tampon phosphate (PBS) et bloqués à l'aide d'une solution PBS-sérum albumine bovine 1 % pendant une heure à 37°C. Les sérums ont été dilués au 1/100^{ème} dans du PBS, introduits en duplicate et incubés pendant une heure à température ambiante. Des anticorps polyclonaux de souris anti-STIP-1 (Tebu-bio, Maryland, USA) dilués au 1/500^{ème} et utilisés comme contrôle positif. Les plaques ont été lavées comme mentionné ci-dessus, et des anticorps de lapin anti-Fcγ humain conjugués à la phosphatase alcaline (Tebu-bio, Maryland, USA; dilués au 1/1000^{ème}), avec des anticorps d'âne anti-IgG de lapin (Jackson ImmunoResearch, West Baltimore Pike, USA; dilués au 1/10000^{ème}) ont été incubés pendant une heure à température ambiante. Les réactivités ont été révélées par ajout de p-nitrophénylphosphate (Sigma-Aldrich, St.Louis, USA) et l'absorbance (DO) à 405 nm a été mesurée. Le bruit de fond de densité optique (puits recouverts de tampon bicarbonate uniquement) a été soustrait de la valeur de DO obtenue avec les protéines. Les échantillons ont été considérés positifs lorsque la densité optique était supérieure ou égale à la moyenne + 2 écarts-types du contrôle (2et).

### Etude de l'effet des sérums ou des IgG purifiées de patients vs sujets sains sur la contraction de CMLV ou de fibroblastes

### Sérums et IgG purifiées

Les inventeurs ont utilisé les sérums de 10 patients sclérodermiques sans HTAP (appelés par la suite ScS), 10 patients sclérodermiques avec HTAP (ScS-HTAP), et 10 patients atteints d'HTAPi. Dix sujets sains appariés pour le sexe et l'âge ont également été testés. Les patients sclérodermiques répondent aux critères de l'American Rheumatology Association (ARA) et/ou aux critères de Leroy et Medsger. Les sérums étaient conservés à -80°C avant leur utilisation. Tous les patients et les sujets sains ont signé un consentement éclairé dans le cadre de l'étude HTAP-Ig (Contrat d'Investigation et de Recherche Clinique 2005 N° CR C 05066, promoteur Assistance Publique-Hôpitaux de Paris; investigateur coordonnateur Luc Mouthon; centre de gestion URC Cochin).

Les IgG ont été purifiées du sérum des patients et des sujets sains sur une colonne de protéine G sépharose. Les IgG purifiées ont été quantifiées par spectrophotométrie à 260 et 280 nm. La pureté des préparations d'IgG purifiées a été attestée par SDS-PAGE.

### Culture cellulaire

Des CMLV humaines obtenues à partir d'artères mammaires chez des patients ayant subi un pontage aorto-coronaire immortalisées après transduction séquentielle lentivirale de la sous-unité catalytique de l'holoenzyme humaine Telomerase Reverse Transcriptase (hTERT) et de l'antigène T du polyomavirus SV40 (Simian Virus 40) dans une culture primaire de CMLV adultes (Weksler et al. 2005) ont été fournies par le Dr Babett Weksler (Institut Cochin, Paris). Ces cellules ont été cultivées dans du milieu de culture DMEM (Gibco BRL Invitrogen™ Cergy Pontoise, France) supplémenté avec 10% de sérum de veau foetal (SVF) filtré et décomplémenté, avec 1% de pénicilline/streptomycine et 1% de ciprofloxacine.

Des CMLV humaines d'aorte (PromoCell, Heidelberg, Allemagne) ont été cultivées dans du milieu de culture Smooth Muscle Cell Growth Medium 2 (PromoCell, Heidelberg, Allemagne) supplémenté avec 5 % de SVF décomplémenté, 0,5 ng/ml d'Epithelial Growth Factor, (EGF), 2 ng/ml de basic Fibroblast Growth Factor (bFGF), 5 µg/ml d'insuline, 1% de pénicilline/streptomycine et 1% de ciprofloxacine.

### Contraction d'une matrice de collagène

Les CMLV ont été récoltées avec de la trypsine 0.25% EDTA 1mM (Gibco BRL Invitrogen™ Cergy Pontoise, France) neutralisée par 5% de SVF. Les matrices de collagène ont été préparées dans des boîtes 35mm avec 1 ml de milieu sans SVF contenant 500 000 CMLV, 1.65 ml de milieu 1% de sérum (SVF, sérum de patient ou sérum sain) ou 128 µg/ml d'IgG purifiés, et 1 ml de collagène 3.35 mg/ml (BD Biosciences, Franklin Lakes, EU). Pour le test de contraction avec activation des CMLV, 40 ng/ml de TNF-α (R&D systems, Abingdon, England) ont été ajoutés. Après une incubation 1h à 37°C permettant la polymérisation, les matrices ont été décollées en tapotant doucement sur les bords de la boîte, afin d'initier la contraction. Pour déterminer le degré de contraction du gel, des photographies ont été effectuées à J2 et J4, afin de mesurer la surface de la matrice. Les résultats obtenus avec les sérums de sujets sains et de patients ont été comparés. Vingt sérums ont été testés dans chaque expérience; les différents tests de contraction ont été étalonnés grâce au SVF et à un sérum de référence utilisé en duplicate. Ce contrôle permettait de vérifier la bonne reproductibilité du test. Les mesures ont été effectuées grâce au logiciel Image J (National Institute of Health NIH, EU).

### Résultants

### Immunoblot 1 D

Dans un premier temps, les inventeurs ont testé séparément les réactivités IgG des sérums de 15 patients dans chaque groupe et de 15 sujets sains en immunoblot 1 D à une dilution de 1/100. Ils ont mis en évidence de nombreuses réactivités avec les sérums de patients (ScS, HTAP-ScS, HTAPi) dont certaines étaient très intenses en comparaison avec les sérums de sujets sains qui ne présentaient quasiment aucune bande d'immunoréactivité IgG. Le nombre des bandes de réactivité était plus important dans le cas des patients sclérodermiques avec ou sans HTAP que dans le cas des patients ayant une HTAPi. De plus, certaines bandes de réactivités semblaient spécifiques d'un groupe de malades donné, en particulier une bande à environ 90 kDa chez certains patients sclérodermiques avec ou sans HTAP **(****Figure 1****).** Afin d'identifier les cibles antigéniques des IgG anti-CMVL des patients, les inventeurs ont ensuite effectué des immunoblots 2 D.

### Cartographie des protéines de CMLV après séparation en deux dimensions

Après avoir fait migrer 100 µg d'extraits protéiques de CMLV préparés comme décrit dans la section Matériels et Méthodes, les inventeurs ont pu séparer puis colorer au nitrate d'argent 880 taches protéiques et obtenir le gel représenté en **figure 2****.** Les inventeurs ont pu estimer le PM et le pHi de chacune de ces tâches protéiques après analyse informatique selon leur disposition dans le gel et à l'aide de marqueurs de PM; elles avaient majoritairement un PM compris entre 10 et 125 kDa et un pHi compris entre 3 et 8.

### Immunoblot 2 D des IgG sériques de sujets sains et de patients vis-à-vis de protéines de cellules musculaires lisses vasculaires

635 réactivités ont été identifiées après appariement des réactivités présentes sur chacune des seize membranes de PVDF avec le gel de référence, en considérant les réactivités des IgG sériques des pools de trois patients et celles du pool de sujets sains additionnées.

### Sujets sains

Les sérums de 12 sujets sains ont été mélangés et leurs réactivités ont été testées. Les IgG de ces sujets reconnaissaient 150 taches protéiques de CMVL **(****Figure 3****).** Vingt et une taches protéiques étaient spécifiques des sujets sains (non reconnues par les patients).

### Patients sclérodermiques

Les 5 pools de 3 sérums de patients atteints de ScS sans HTAP reconnaissaient en moyenne 127±26 taches protéiques **(****figure 3****)** et un total de 367 taches différentes. 71 % de ces taches n'étaient pas reconnues par les sujets sains. Parmi ces 367 taches, 13 étaient communes aux 5 pools de patients sclérodermiques (dont une seule non reconnue par les sujets sains), 18 communes à 4 pools sur 5 (dont 7 non reconnues par les sujets sains) et 39 à 3 pools sur 5 (dont 19 non reconnues par les sujets sains) **(****figure 4****).**

Les taches protéiques communes aux 5 pools de patients ScS sans HTAP étaient également toutes reconnues par certains pools de patients atteints d'HTAPi et d'HTAP-ScS. Sur les 18 taches protéiques reconnues par 4/5 des pools de patients ScS, 9 étaient également reconnues par au moins 3/5 des pools de patients de chacun des deux autres groupes de malades (dont 3 non reconnues par les sujets sains), 5 étaient reconnues par au moins 3/5 des pools des patients HTAP-ScS (dont une non reconnue par les sujets sains), et 3 étaient reconnues par au moins 3/5 des pools des patients HTAPi (dont 2 non reconnues par les sujets sains). Une tache (5325) était reconnue par un seul pool de patients HTAP-ScS, par aucun pool de patients HTAPi et par aucun pool de sujets sains.

Les IgG des 5 pools de 3 sérums de patients atteints d'HTAP-ScS reconnaissaient en moyenne 145±48 taches protéiques **(****figure 4****).** Au total, 264 taches protéiques différentes étaient reconnues par les IgG sériques de ces patients, dont 77 % non reconnues part les IgG des sujets sains. Parmi ces 264 taches protéiques, 19 étaient communes aux 5 pools de patients HTAP-ScS (dont 2 non reconnues par les sujets sains), 29 communes à 4/5 des pools (dont 9 non reconnues par les sujets sains) et 47 à 3/5 des pools (dont 30 non reconnues par les sujets sains) **(****figure 4****).**

Les taches protéiques communes aux 5 pools de patients HTAP-ScS étaient également majoritairement reconnues par les patients atteints d'HTAPi et d'HTAP-ScS. Plus précisément, 16 taches étaient reconnues par au moins 3/5 des pools de patients des deux autres groupes (dont 1 seule non reconnue par les sujets sains), 3 étaient reconnues par au moins 3/5 des pools de patients HTAPi (dont 1 seule non reconnue par les sujets sains) et 1 tache était reconnue par au moins 3/5 des pools de patients ScS.

Contrairement aux patients atteints de ScS, la majorité des taches reconnues par 4/5 des pools de patients HTAP-ScS étaient reconnues par moins de 40 % des malades des deux autres groupes. Plus précisément, 12 taches étaient reconnues par moins de 40 % des malades des deux autres groupes, dont 5 taches non reconnues par les patients ScS (4658, 5206, 4831, 4707, 4659) et 3 taches non reconnues par les patients HTAPi (4656, 5190, 4707). 9 taches étaient reconnues par au moins 3/5 des pools des patients ScS et HTAPi (dont 2 non reconnues par les sujets sains), 5 étaient reconnues par au moins 3/5 des patients HTAPi (dont 1 non reconnue par les sujets sains) et 3 étaient reconnues par au moins 3/5 des patients ScS (ces 3 taches étaient également toutes reconnues par les sujets sains).

### Patients atteints d'HTAP idiopathique

Les IgG des 5 pools de 3 sérums de patients atteints d'HTAPi reconnaissaient en moyenne 130±25 taches protéiques **(****figure 3****).** Au total, 356 taches protéiques différentes étaient reconnues par les IgG de ces patients, et 70 % n'étaient pas reconnues part les IgG des sujets sains. Parmi ces 356 taches protéiques, 12 étaient communes aux 5 pools de patients (mais toutes étaient reconnues par les sujets sains), 24 communes à 4/5 des pools (dont 7 non reconnues par les sujets sains) et 54 à 3/5 des pools (dont 31 non reconnues par les sujets sains) **(****figure 4****).**

Les taches protéiques communes aux 5 pools de patients HTAPi étaient également majoritairement reconnues par des pools de patients atteints d'HTAPi ou d'HTAP-ScS. Plus précisément, 10 étaient reconnues également par au moins 3/5 des pools de patients ScS et de patients HTAP-ScS, une était reconnue également par au moins 3/5 des pools des patients ScS et une autre par au moins 3/5 des pools des patients HTAP-ScS.

Les taches protéiques reconnues par 4/5 des pools de sérums de patients HTAPi étaient majoritairement partagées avec les 2 autres groupes de malades. Plus précisément, 15 taches étaient reconnues également par au moins 3/5 des pools des patients ScS et 3/5 des pools des patients HTAP-ScS (dont 4 non reconnues par les sujets sains), 3 taches étaient également reconnues par au moins 3/5 des pools des patients ScS (dont une non reconnue par les sujets sains) et une était reconnue aussi par au moins 3/5 des pools des patients HTAP-ScS (et par les sujets sains). 5 taches étaient reconnues par moins de 40 % des pools des patients ScS ou HTAP-ScS (dont 4 non reconnues par les sujets sains). Parmi ces 4 taches, une n'était pas reconnue par les patients ScS (4735).

### Comparaison des réactivités des IgG de patients sclérodermiques avec ou sans HTAP, de patients ayant une HTAP idiopathique et de sujets sains et identification des antigènes spécifiques d'un groupe de malades

Les inventeurs ont comparé les profils de réactivité des IgG du pool de sérums de sujets sains et des pools de sérums de patients vis-à-vis des protéines de CMLV. Quel que soit le groupe de patients, la plupart des taches protéiques non reconnues par les IgG de sujets sains l'étaient par un seul pool de patient sur 5 **(****figure 5****).** En sélectionnant les taches protéiques reconnues par au moins 3/5 des pools de sérums de patients d'un groupe donné et non pas par le pool des sujets sains, les inventeurs ont identifié 21 taches protéiques d'intérêt **(tableau 1).** Même si le résultat de toutes les taches protéiques digérées n'a pas encore été obtenu, 13 taches protéiques intéressantes ont pu être identifiées. La localisation de ces taches protéiques sur le gel de référence est indiquée dans la **figure 6****.** Deux taches (5190, 5325) semblent spécifiques de la ScS puisqu'elles sont reconnues respectivement par 3/5 et 4/5 des pools de patients ScS et 4/5 et 1/5 des pools de patients HTAP-ScS, mais par aucun pool de patients HTAPi. L'une d'entre elle (5325) a été identifiée comme étant la galectine.

**Tableau 1 : Identification des taches protéiques reconnues par les IgG d'au moins 4/5 des pools de patients d'un groupe donné et non par les IgG du pool de sujets sains. L'identification d'un même antigène candidat pour des spots différents correspond à la détection d'isoformes de la protéine.**

| **Spot** | **pHi** | **PM(kDa)** | **antigène candidat** | **nombre de pools de patients reconnaissant l'antigène** | | | **Nom et numéro d'accès Swissprot des antigènes candidats** |
|---|---|---|---|---|---|---|---|
| | | | | **ScS (n=5)** | **HTAP-ScS (n=5)** | **HTAPi (n=5)** | |
| 4484 | 6.7 | 82 | Précurseur de la protéine de 78 kDa régulée par le glucose | 4 | 0 | 3 | GRP78_HUMAN |
| | | | | | | | **P11021** |
| | | | | | | | (SEQ ID NO:18) |
| 4488 | 6.8 | 81 | Caldesmone | 2 | 2 | 4 | CALD1_HUMAN |
| | | | | | | | **Q05682** |
| | | | | | | | (SEQ ID NO:5) |
| 4735 | 5.5 | 51 | Protéine FAM10A4 | 0 | 2 | 4 | F10A4_HUMAN |
| | | | | | | | **Q8IZP2** |
| | | | | | | | (SEQ ID NO:6) |
| 4787 | 5.7 | 46 | Actine cytoplasmique 2 | 3 | 3 | 4 | ACTG_HUMAN |
| | | | | | | | **P63261** |
| | | | | | | | (SEQ ID NO:8) |
| 4660 | 6.2 | 60 | Précurseur A3 de la protéine disulfide-isomérase, Desmine, | 1 | 4 | 1 | PDIA3_HUMAN |
| | | | | | | | **P30101** |
| | | | | | | | (SEQ ID NO:10) |
| | | | | | | | DESM_HUMAN |
| | | | | | | | **P17661** |
| | | | | | | | (SEQ ID NO:11) |
| | | | Périphérine | | | | PERI_HUMAN |
| | | | | | | | **P41219** |
| | | | | | | | (SEQ ID NO:12) |
| 4691 | 6.4 | 56 | Ribonucléoprotéine H nucléaire hétérogène | 1 | 4 | 1 | HNRH1_HUMAN |
| | | | | | | | **P31943** |
| | | | | | | | (SEQ ID NO:13) |

### Identification des antigènes cibles des IgG anti-CMLV reconnus avec une intensité significativement plus forte chez les malades que chez les sujets sains

Dans un deuxième temps les inventeurs ont d'identifié les taches protéiques de CMLV reconnues par les IgG de pools de 3 sérums de patients et par les IgG du pool de sujets sains, à la condition que ces taches protéiques soient reconnues avec une forte intensité par les IgG d'un grand nombre de pools de 3 sérums de patients et avec une plus forte intensité que les sujets sains. Vingt sept taches protéiques ont répondu à ces critères **(Tableau 2).** Les réactivités des IgG sériques des différents groupes de malades vis-à-vis des taches protéiques 4576, 4570 et 4576 identifiées comme des isoformes de la phosphoprotéine induite par le stress et vis-à-vis de la tache 4738 identifiée comme l'α-énolase sont représentées sur la **figure 7****.** La région sélectionnée est représentée sur la **figure 6****.**

**Tableau 2 : Identification des taches protéiques reconnues avec une intensité significativement plus forte chez les patients que chez les sujets sains**

| **Spot** | **pHi** | **PM(kDa)** | **antigène candidat** | **nombre de pools de patients reconnaissant l'antigène** | | | **Nom et numéro d'accès Swissprot des antigènes candidats** |
|---|---|---|---|---|---|---|---|
| | | | | **ScS (n=5)** | **HTAPi (n=5)** | **HTAP-ScS (n=5)** | |
| 4757 | 5.2 | 49 | Vimentine | 5 | 4 | 5 | VIME_HUMAN |
| | | | | | | | **P08670** |
| | | | | | | | (SEQ ID NO:19) |
| 4576 | 6.9 | 70 | Phosphoprotéine 1 induite par le stress | 5 | 4 | 5 | STIP1_HUMAN |
| | | | | | | | **P31948** |
| | | | | | | | (SEQ ID NO:14) |
| 4570 | 7.1 | 70 | Phosphoprotéine 1 induite par le stress | 5 | 5 | 5 | STIP1_HUMAN |
| | | | | | | | **P31948** |
| | | | | | | | (SEQ ID NO:14) |
| 4575 | 6.8 | 70 | Phosphoprotéine 1 induite par le stress | 5 | 4 | 5 | STIP1_HUMAN |
| | | | | | | | **P31948** |
| | | | | | | | (SEQ ID NO:14) |
| 4738 | 7.4 | 51 | α-énolase | 5 | 5 | 5 | ENOA_HUMAN |
| | | | | | | | **P06733** |
| | | | | | | | (SEQ ID NO:20) |
| 5052 | 6.7 | 27 | Triosephosphate isomérase | 3 | 2 | 2 | TPIS_HUMAN |
| | | | | | | | **P60174** |
| | | | | | | | (SEQ ID NO:15) |
| 4536 | 6.1 | 74 | Précurseur de l'albumine sérique | 4 | 4 | 4 | ALBU_HUMAN |
| | | | | | | | **P02768** |
| | | | | | | | (SEQ ID NO:1) |
| 5063 | 5.8 | 26 | Isozyme L1 carboxyl-terminal hydrolase ubiquitine | 4 | 1 | 3 | UCHL1_HUMAN |
| | | | | | | | **P09936** |
| | | | | | | | (SEQ ID NO:4) |
| 5064 | 5.9 | 26 | Isozyme L1 carboxyl-terminal hydrolase ubiquitine | 4 | 1 | 4 | UCHL1_HUMAN |
| | | | | | | | **P09936** |
| | | | | | | | (SEQ ID NO:4) |
| 4463 | 6.7 | 85 | Zyxine | 4 | 3 | 4 | ZYX_HUMAN |
| | | | | | | | **Q15942** |
| | | | | | | | (SEQ ID NO:2) |
| 4539 | 6.2 | 73 | Précurseur de l'albumine sérique | 4 | 4 | 4 | ALBU_HUMAN |
| | | | | | | | **P02768** |
| | | | | | | | (SEQ ID NO:1) |
| 5325 | 5.4 | 15 | Galectine-1 | 4 | 0 | 1 | LEG1_HUMAN |
| | | | | | | | **P09382** |
| | | | | | | | (SEQ ID NO:3) |
| 4734 | 7.0 | 51 | α-énolase | 4 | 1 | 5 | ENOA_HUMAN |
| | | | | | | | **P06733** |
| | | | | | | | (SEQ ID NO:20) |
| 5047 | 6.8 | 27 | Peroxirédoxine-6 | 2 | 5 | 4 | PRDX6_HUMAN |
| | | | | | | | **P30041** |
| | | | | | | | (SEQ ID NO:16) |
| 4441 | 7.4 | 89 | Protéine 2 (Far upstream element-binding protein 2) | 3 | 4 | 3 | FUBP2_HUMAN |
| | | | | | | | **Q92945** |
| | | | | | | | (SEQ ID NO:7) |
| 4446 | 7.2 | 89 | Protéine 2 (Far upstream element-binding protein 2) | 3 | 4 | 2 | FUBP2_HUMAN |
| | | | | | | | **Q92945** |
| | | | | | | | (SEQ ID NO:7) |
| 4833 | 4.9 | 43 | Précurseur de la réticulocalbine-1 | 2 | 3 | 5 | RCN1_HUMAN |
| | | | | | | | **Q15293** |
| | | | | | | | (SEQ ID NO:17) |
| 4747 | 5.2 | 50 | γ-énolase, | 1 | 3 | 5 | ENOG_HUMAN |
| | | | | | | | **P09104** |
| | | | | | | | (SEQ ID NO:9) |
| | | | Vimentine | | | | VIME_HUMAN |
| | | | | | | | **P08670** |
| | | | | | | | (SEQ ID NO:19) |

### Dosage ELISA d'anticorps anti-STIP1

Les inventeurs ont mis en évidence que 56/75 (74.6%) patients sclérodermiques, 24/74 (32.4%) patients ayant une HTAPi, 27/37 (73%) patients ayant une HTAP-SSc et 2/70 (2.8%) sujets sains avaient des anticorps anti-STIP1. Ainsi près de trois quarts des patients sclérodermiques, qu'ils aient ou non une HTAP et près d'un tiers des patients atteints d'HTAPi avaient des Ac anti-STIP1, tandis que ces anticorps étaient en règle générale absents chez les sujets sains.

### Effet des sérums ou des IgG purifiées de patients vs sujets sains sur la contraction de CMLV ou de fibroblastes

Les inventeurs ont déterminé si les sérums et/ou les IgG sériques de patients ayant une ScS et/ou une HTAP avaient un effet sur la contraction des CMLV et des fibroblastes, phénomène impliqué dans le remodelage vasculaire et la mobilité des cellules. Pour cela, les cellules ont été ensemencées dans une matrice de collagène, et incubées en présence de 1% de SVF, de sérums, ou d'IgG purifiées de patients ayant une ScS et/ou une HTAP versus ceux de sujets sains. La rétraction quantifiable de la matrice de collagène reflète l'activité contractile des cellules. L'expérience a été réalisée à partir de cellules saines et non activées. La cinétique de contraction des matrices de collagène a été suivie pendant 4 jours pour les CMLV et 7 jours pour les fibroblastes. Les résultats obtenus avec les CMLV sont présentés dans la figure 9.

### CMLV

Pour ces cellules, les sérums de 15 patients de chaque condition pathologique (ScS, HTAPi, ScS-HTAP) et de 15 sujets sains ainsi que les IgG purifiées de 10 de ces 15 patients et de 10 de ces 15 sujets sains ont été testés. Le SVF utilisé en duplicate, a permis de pondérer les différents tests entre eux. La cinétique de contraction des matrices de collagène a été suivie pendant 4 jours, des expériences préliminaires ayant mis en évidence que les modifications observées au-delà étaient minimes (Fig. 9B) ; les surfaces des matrices de collagène ont été mesurées à J2 et J4 à l'aide du logiciel Image J et calculées en pourcentage de la surface de la matrice initiale.

A J4, la moyenne des surfaces des 15 matrices (en % de la surface initiale) incubées avec le sérum était de 27,8%±6,0 pour les ScS, 31,1%±8,3 pour les HTAPi, 29,4%±4,7 pour les ScS-HTAP, et 34,3%±7,1 pour les sujets sains. Les 15 surfaces des matrices de collagène incubées avec le sérum des patients ScS différaient significativement comparativement aux surfaces des 15 matrices incubées avec le sérum des sujets sains (p = 0,012). En revanche, les différences entre les surfaces obtenues en présence des deux autres groupes de sérums de patients (HTAPi, ScS-HTAP) et le groupe de sérums des sujets sains n'étaient pas significatives.

A J4, la moyenne des surfaces des 10 matrices (en % de la surface initiale) incubées avec les IgG purifiées était de 53,9%±8,2 pour les ScS, 48,0%±3,2 pour les HTAPi, 55,8%±8,9 pour les ScS-HTAP, et 34,3%±8,6 pour les sujets sains. On note une différence significative entre les matrices incubées avec les IgG purifiées de patients HTAPi et celles incubées avec les IgG de sujets sains (p = 0,001).

Si on compare les deux expériences entre elles (Fig.9B1 vs 9B2), on remarque que les matrices incubées avec les IgG purifiées se rétractaient moins que celles incubées avec les sérums.

### Exemple 2

Les inventeurs ont soumis les échantillons des patients atteints d'une ScS, d'une HTAP-ScS ou d'une HTAPi, ainsi que les échantillons de sujets sains, à une autre analyse de leurs réactivités afin d'affiner les résultats obtenus et identifier d'autres anticorps anti-CMLV.

Cette étude a permis de mettre en évidence des réactivités contre la Péroxyrédoxine-2 (spot 5122; Swissprot: PRDX2_HUMAN, n°P32119; SEQ ID NO:21), le précurseur mitochondrial de la péroxyde réductase dépendant de la thiorédoxine (Thioredoxin-dependent peroxide reductase, mitochondrial precursor; spot 5096; Swissprot: PRDX3_HUMAN, n°P30048; SEQ ID NO:22), de la protéine d'activation de GTPase spécifique de Ran (Ran-specific GTPase-activating protein; spot 5024; Swissprot: RANG_HUMAN, n°P43487; SEQ ID NO:23) et de la High mobility group protein B1 (spot 5011; Swissprot: HMGB1_HUMAN, n°P09429; SEQ ID NO:24). Des réactivités contre la chaîne bêta de la tubuline et contre la polymerase I and transcript release factor dans le spot 4672. Parmi ces réactivités, celles dirigées contre la péroxyrédoxine-2, la chaîne bêta de la tubuline et la polymerase I and transcript release factor sont spécifiquement présentes dans les IgG des pools de patients, et non dans les IgG du pool de sujets sains. Les réactivités contre le précurseur mitochondrial de la péroxyde réductase dépendant de la thiorédoxine, la protéine d'activation de GTPase spécifique de Ran et la High mobility group protein B1 ont été identifiées dans les pools de patients et de sujets sains, mais de manière significativement plus élevée chez les patients.

De plus, les résultats présentés dans l'exemple 1 ont pu être affinés. Les inventeurs ont ainsi pu montrer que les anticorps anti-actine cytoplasmique 2 sont présents dans les pools de patients et de sujets sains, mais de manière significativement plus élevées chez les patients. Ils ont également pu montrer l'existence de réactivité contre la galectine 1 et la zyxine dans les IgG des pools de patients, et non dans les IgG du pool de sujets sains.

### Références

Bordron et al, 1998, Arthritis and rheumatism, 41(10):1738-47.
Chizzolini et al, 2002, Arthritis and rheumatism, 46(6):1602-13.
Dorfmuller et al, 2003, Eur Respir J, 22(2) :358-63
Garcia de la Pena-Lefebvre et al, 2004, Clin Immunol, 111(3):241-51.
Hachulla et al, 2005, Arthritis and rheumatism, 52(12):3792-800.
Henault et al, 2006, Arthritis and rheumatism, 54(3):963-73.
Moroi et al, 1980, Proceedings of the National Academy of Sciences of the United States of America, 77(3):1627-31.
Mouthon et al, 2005, Eur Respir J, 26(6) :986-8
Nicolls MR et al, 2005, Eur Respir J, 26(6):1110-8.
Rabilloud et al, 1990, Electrophoresis, 11(10):785-94.
Rubin, 1997, N Engl J Med, 336(2) :111-7.
Servettaz et al, Clinical immunology, 120(2):212-9.
Tamby et al, 2005, Thorax 60(9):765-72
Tamby et al, 2006, Eur Respir J, 28(4):799-807.
Tamby et al, 2007, Annals of the New York Academy of Sciences, 1109:221-8.
Terrier et al, 2008, Am J Respir Crit Care Med, 177:1128-1134. 30 Weksler et al, 2005 Faseb J, 19(13):1872-4.

## Revendications

1. Procédé *in vitro* de détection d'une sclérodermie systémique (ScS) ou d'une hypertension artérielle pulmonaire associée à une sclérodermie (HTAP-ScS) ou d'une hypertension artérielle pulmonaire idiopathique (HTAPi) chez un sujet, comprenant la détermination de la présence et/ou de la quantité d'au moins un anticorps choisi dans le groupe constitué des anticorps anti-Galectine-1, anti-protéine FAM10A4, anti-phosphoprotéine 1 induite par le stress, et anti-précurseur de la protéine de 78 kDa régulée par le glucose,
dans un échantillon biologique provenant d'un patient,
la présence d'un anticorps anti-précurseur de la protéine de 78 kDa régulée par le glucose étant indicative d'une ScS ou d'une HTAPi ;
la présence d'un anticorps anti-protéine FAM10A4 étant indicative d'une HTAP-ScS ou d'une HTAPi ;
la présence d'un anticorps anti-galectine-1 étant indicative d'une ScS ;
ou la présence en une quantité supérieure à une valeur contrôle d'un anticorps anti-phosphoprotéine 1 induite par le stress étant indicative d'une ScS, d'une HTAP-ScS ou d'une HTAPi.

2. Procédé selon la revendication 1, dans lequel l'échantillon biologique est un échantillon de sang ou de sérum.

3. Procédé selon l'une des revendications 1 ou 2, dans lequel la quantité dudit au moins un anticorps est déterminée par un immunoessai.

4. Procédé selon la revendication 3, dans lequel l'immunoessai est un dosage ELISA.

5. Procédé selon l'une des revendications 1 à 4, dans lequel le patient est un humain.

6. Procédé selon l'une des revendications 1 à 5, dans lequel le patient souffre d'une sclérodermie systémique, avec ou sans hypertension artérielle pulmonaire associée.

7. Procédé selon l'une des revendications 1 à 5, dans lequel le patient souffre d'une hypertension artérielle pulmonaire idiopathique.

8. Procédé selon l'une des revendications 1 à 6, dans lequel l'hypertension artérielle pulmonaire est associée à une hypertension portale, à une cardiopathie congénitale, ou à une infection par le virus de l'immunodéficience humaine (VIH), ou est une hypertension pulmonaire post-embolique.

9. Procédé selon l'une des revendications 1 à 6, dans lequel le patient est un sujet prédisposé à développer une sclérodermie systémique ou une hypertension artérielle pulmonaire associée à une sclérodermie ou une hypertension artérielle pulmonaire idiopathique.

10. Procédé selon la revendication 9, dans lequel le sujet est porteur d'une ou plusieurs mutation(s) dans le gène codant BMPRII, endoglin ou ALK1.

11. Procédé selon l'une quelconque des revendications 1 à 10, ledit au moins un anticorps comprenant un anticorps anti-galectine 1.

12. Procédé selon l'une quelconque des revendications 1 à 10, ledit au moins un anticorps comprenant un anticorps anti-phosphoprotéine 1 induite par le stress.

## Patentansprüche

1. *In vitro* Verfahren zum Nachweis einer systemischen Sklerodermie (ScS) oder einer pulmonal-arteriellen Hypertonie, die mit einer Sklerodermie assoziiert ist (HTAP-ScS), oder einer idiopathischen pulmonal-arteriellen Hypertonie (HTAPi) bei einer Person, umfassend die Bestimmung des Vorhandenseins und/oder der Menge wenigstens eines Antikörpers, der aus der Gruppe ausgewählt ist, bestehend aus Anti-Galectin-1-Antikörper, Anti-FAM10A4-Protein-Antikörper, Anti-Stress-induziertes Phosphoprotein 1-Antikörper und Anti-Precursor-Antikörper des Glucose-regulierten 78 kDa Proteins,
in einer von einem Patienten stammenden biologischen Probe,
wobei das Vorhandensein eines Anti-Precursor-Antikörpers des Glucose-regulierten 78 kDa Proteins ein Hinweis auf eine ScS oder eine HTAPi ist;
wobei das Vorhandensein eines Anti-FAM10A4-Protein-Antikörpers ein Hinweis auf eine HTAP-ScS oder eine HTAPi ist;
wobei das Vorhandensein eines Anti-Galectin-1-Antikörpers ein Hinweis auf eine ScS ist;
oder wobei das Vorhandensein in einer größeren Menge als ein Kontrollwert eines Anti-Stress-induziertes Phosphoprotein 1-Antiköipers ein Hinweis auf eine ScS, eine HTAP-ScS oder eine HTAPi ist.

2. Verfahren gemäß Anspruch 1, wobei die biologische Probe eine Blut- oder Serumprobe ist.

3. Verfahren gemäß einem der Ansprüche 1 oder 2, wobei die Menge des besagten wenigstens einen Antikörpers mit einem Immunassay bestimmt wird.

4. Verfahren gemäß Anspruch 3, wobei der Immunassay ein ELISA ist.

5. Verfahren gemäß einem der Ansprüche 1 bis 4, wobei der Patient ein Mensch ist.

6. Verfahren gemäß einem der Ansprüche 1 bis 5, wobei der Patient an einer systemischen Sklerodermie mit oder ohne assoziierte pulmonal-arterielle Hypertonie leidet.

7. Verfahren gemäß einem der Ansprüche 1 bis 5, wobei der Patient an einer idiopathischen pulmonal-arteriellen Hypertonie leidet.

8. Verfahren gemäß einem der Ansprüche 1 bis 6, wobei die pulmonal-arterielle Hypertonie mit einer portalen Hypertonie, mit einer kongenitalen Kardiopathie oder mit einer Infektion mit dem humanen Immunschwäche-Virus (HIV) assoziiert ist, oder eine postembolische pulmonale Hypertonie ist.

9. Verfahren gemäß einem der Ansprüche 1 bis 6, wobei der Patient eine Person ist, die für die Entwicklung einer systemischen Sklerodermie oder einer mit einer Sklerodermie assoziierten pulmonal-arteriellen Hypertonie oder einer idiopathischen pulmonal-arteriellen Hypertonie prädisponiert ist.

10. Verfahren gemäß Anspruch 9, wobei die Person Trägerin einer oder mehrerer Mutationen im BMPRII, Endoglin oder ALK1 codierenden Gen ist.

11. Verfahren gemäß einem der Ansprüche 1 bis 10, wobei besagter wenigstens eine Antikörper einen Anti-Galectin 1-Antikörper umfasst.

12. Verfahren gemäß einem der Ansprüche 1 bis 10, wobei besagter wenigstens eine Antikörper einen Anti-Stress-induziertes Phosphoprotein 1-Antikörper umfasst.

## Claims

1. An *in vitro* process for detecting systemic scleroderma (SSc) or pulmonary arterial hypertension associated with scleroderma (PAH-SSc) or idiopathic pulmonary arterial hypertension (IPAH) in a subject, comprising the determination of the presence and/or amount of at least one antibody selected from the group of antibodies formed by anti-Galectin-1, anti-protein FAM10A4, stress-induced anti-phosphoprotein 1 and anti-precursor of the 78 kDA glucose-regulated protein,
in a biological sample taken from a patient,
the presence of an anti-precursor antibody of the 78 kDa glucose-regulated protein being indicative of SSc or IPAH;
the presence of an anti-protein FAM10A4 antibody being indicative of PAH-SSc or IPAH;
the presence of an anti-Galectin-1 antibody being indicative of SSc;
or the presence in an amount higher than a control value of stress-induced anti-phosphoprotein 1 antibody being indicative of SSc, PAH-SSc or IPAH.

2. The process according to claim 1, wherein the biological sample is a blood or serum sample.

3. The process according to one of claims 1 or 2, wherein the amount of said at least one antibody is determined by immunoassay.

4. The process according to claim 3, wherein the immunoassay is an ELISA assay.

5. The process according to one of claims 1 to 4, wherein the patient is a human.

6. The process according to one of claims 1 to 5, wherein the patient suffers from systemic scleroderma, with or without associated pulmonary arterial hypertension.

7. The process according to one of claims 1 to 5, wherein the patient suffers from idiopathic pulmonary arterial hypertension.

8. The process according to one of claims 1 to 6, wherein the pulmonary arterial hypertension is associated with portal hypertension, congenital heart disease, or infection with human immunodeficiency virus (HIV) or is post-embolic pulmonary hypertension.

9. The process according to one of claims 1 to 6, wherein the patient is a subject predisposed to developing systemic scleroderma, or pulmonary arterial hypertension associated with scleroderma, or idiopathic pulmonary arterial hypertension.

10. The process according to claim 9, wherein the subject is a carrier of one or more mutations in the gene encoding BMPRII, endoglin or ALK1.

11. The process according to any of claims 1 to 10, the said at least one antibody comprising an anti-Galectin 1 antibody.

12. The process according to any of claims 1 to 10, the said at least one antibody comprising a stress-induced anti-phosphoprotein 1 antibody.


### SEQUENCE LISTING

<110> ASSISTANCE PUBLIQUE - HOPITAUX DE PARIS UNIVERSITE RENE DESCARTES - PARIS 5 UNIVERSITE PARIS-SUD 11
<120> Procédé de diagnostic d'une sclérodermie systémique ou d'une hypertension artérielle pulmonaire
<130> B762PC00
<160> 24
<170> PatentIn version 3.3
<210> 1
   <211> 609
   <212> PRT
   <213> Homo sapiens
<400> 1
<210> 2
   <211> 572
   <212> PRT
   <213> Homo sapiens
<400> 2
   Met Ala Ala Pro Arg Pro Ser Pro Ala Ile Ser Val Ser Val Ser Ala
   1               5                   10                  15
   Pro Ala Phe Tyr Ala Pro Gln Lys Lys Phe Gly Pro Val Val Ala Pro
               20                  25                  30
   Lys Pro Lys Val Asn Pro Phe Arg Pro Gly Asp Ser Glu Pro Pro Pro
           35                  40                  45
   Ala Pro Gly Ala Gln Arg Ala Gln Met Gly Arg Val Gly Glu Ile Pro
       50                  55                  60
   Pro Pro Pro Pro Glu Asp Phe Pro Leu Pro Pro Pro Pro Leu Ala Gly
   65                  70                  75                  80
   Asp Gly Asp Asp Ala Glu Gly Ala Leu Gly Gly Ala Phe Pro Pro Pro
                   85                  90                  95
   Pro Pro Pro Ile Glu Glu Ser Phe Pro Pro Ala Pro Leu Glu Glu Glu
               100                 105                 110
   Ile Phe Pro Ser Pro Pro Pro Pro Pro Glu Glu Glu Gly Gly Pro Glu
           115                 120                 125
   Ala Pro Ile Pro Pro Pro Pro Gln Pro Arg Glu Lys Val Ser Ser Ile
       130                 135                 140
   Asp Leu Glu Ile Asp Ser Leu Ser Ser Leu Leu Asp Asp Met Thr Lys
   145                 150                 155                 160
   Asn Asp Pro Phe Lys Ala Arg Val Ser Ser Gly Tyr Val Pro Pro Pro
                   165                 170                 175
   Val Ala Thr Pro Phe Ser Ser Lys Ser Ser Thr Lys Pro Ala Ala Gly
               180                 185                 190
   Gly Thr Ala Pro Leu Pro Pro Trp Lys Ser Pro Ser Ser Ser Gln Pro
           195                 200                 205
   Leu Pro Gln Val Pro Ala Pro Ala Gln Ser Gln Thr Gln Phe His Val
       210                 215                 220
   Gln Pro Gln Pro Gln Pro Lys Pro Gln Val Gln Leu His Val Gln Ser
   225                 230                 235                 240
   Gln Thr Gln Pro Val Ser Leu Ala Asn Thr Gln Pro Arg Gly Pro Pro
                   245                 250                 255
   Ala Ser Ser Pro Ala Pro Ala Pro Lys Phe Ser Pro Val Thr Pro Lys
               260                 265                 270
   Phe Thr Pro Val Ala Ser Lys Phe Ser Pro Gly Ala Pro Gly Gly Ser
           275                 280                 285
   Gly Ser Gln Pro Asn Gln Lys Leu Gly His Pro Glu Ala Leu Ser Ala
       290                 295                 300
   Gly Thr Gly Ser Pro Gln Pro Pro Ser Phe Thr Tyr Ala Gln Gln Arg
   305                 310                 315                 320
   Glu Lys Pro Arg Val Gln Glu Lys Gln His Pro Val Pro Pro Pro Ala
                   325                 330                 335
   Gln Asn Gln Asn Gln Val Arg Ser Pro Gly Ala Pro Gly Pro Leu Thr
               340                 345                 350
   Leu Lys Glu Val Glu Glu Leu Glu Gln Leu Thr Gln Gln Leu Met Gln
           355                 360                 365
   Asp Met Glu His Pro Gln Arg Gln Asn Val Ala Val Asn Glu Leu Cys
       370                 375                 380
   Gly Arg Cys His Gln Pro Leu Ala Arg Ala Gln Pro Ala Val Arg Ala
   385                 390                 395                 400
   Leu Gly Gln Leu Phe His Ile Ala Cys Phe Thr Cys His Gln Cys Ala
                   405                 410                 415
   Gln Gln Leu Gln Gly Gln Gln Phe Tyr Ser Leu Glu Gly Ala Pro Tyr
               420                 425                 430
   Cys Glu Gly Cys Tyr Thr Asp Thr Leu Glu Lys Cys Asn Thr Cys Gly
           435                 440                 445
   Glu Pro Ile Thr Asp Arg Met Leu Arg Ala Thr Gly Lys Ala Tyr His
       450                 455                 460
   Pro His Cys Phe Thr Cys Val Val Cys Ala Arg Pro Leu Glu Gly Thr
   465                 470                 475                 480
   Ser Phe Ile Val Asp Gln Ala Asn Arg Pro His Cys Val Pro Asp Tyr
                   485                 490                 495
   His Lys Gln Tyr Ala Pro Arg Cys Ser Val Cys Ser Glu Pro Ile Met
               500                 505                 510
   Pro Glu Pro Gly Arg Asp Glu Thr Val Arg Val Val Ala Leu Asp Lys
           515                 520                 525
   Asn Phe His Met Lys Cys Tyr Lys Cys Glu Asp Cys Gly Lys Pro Leu
       530                 535                 540
   Ser Ile Glu Ala Asp Asp Asn Gly Cys Phe Pro Leu Asp Gly His Val
   545                 550                 555                 560
   Leu Cys Arg Lys Cys His Thr Ala Arg Ala Gln Thr
                   565                 570
<210> 3
   <211> 135
   <212> PRT
   <213> Homo sapiens
<400> 3
   Met Ala Cys Gly Leu Val Ala Ser Asn Leu Asn Leu Lys Pro Gly Glu
   1               5                   10                  15
   Cys Leu Arg Val Arg Gly Glu Val Ala Pro Asp Ala Lys Ser Phe Val
               20                  25                  30
   Leu Asn Leu Gly Lys Asp Ser Asn Asn Leu Cys Leu His Phe Asn Pro
           35                  40                  45
   Arg Phe Asn Ala His Gly Asp Ala Asn Thr Ile Val Cys Asn Ser Lys
       50                  55                  60
   Asp Gly Gly Ala Trp Gly Thr Glu Gln Arg Glu Ala Val Phe Pro Phe
   65                  70                  75                  80
   Gln Pro Gly Ser Val Ala Glu Val Cys Ile Thr Phe Asp Gln Ala Asn
                   85                  90                  95
   Leu Thr Val Lys Leu Pro Asp Gly Tyr Glu Phe Lys Phe Pro Asn Arg
               100                 105                 110
   Leu Asn Leu Glu Ala Ile Asn Tyr Met Ala Ala Asp Gly Asp Phe Lys
           115                 120                 125
   Ile Lys Cys Val Ala Phe Asp
       130                 135
<210> 4
   <211> 223
   <212> PRT
   <213> Homo sapiens
<400> 4
<210> 5
   <211> 793
   <212> PRT
   <213> Homo sapiens
<400> 5
   Met Asp Asp Phe Glu Arg Arg Arg Glu Leu Arg Arg Gln Lys Arg Glu
   1               5                   10                  15
   Glu Met Arg Leu Glu Ala Glu Arg Ile Ala Tyr Gln Arg Asn Asp Asp
               20                  25                  30
   Asp Glu Glu Glu Ala Ala Arg Glu Arg Arg Arg Arg Ala Arg Gln Glu
           35                  40                  45
   Arg Leu Arg Gln Lys Gln Glu Glu Glu Ser Leu Gly Gln Val Thr Asp
       50                  55                  60
   Gln Val Glu Val Asn Ala Gln Asn Ser Val Pro Asp Glu Glu Ala Lys
   65                  70                  75                  80
   Thr Thr Thr Thr Asn Thr Gln Val Glu Gly Asp Asp Glu Ala Ala Phe
                   85                  90                  95
   Leu Glu Arg Leu Ala Arg Arg Glu Glu Arg Arg Gln Lys Arg Leu Gln
               100                 105                 110
   Glu Ala Leu Glu Arg Gln Lys Glu Phe Asp Pro Thr Ile Thr Asp Ala
           115                 120                 125
   Ser Leu Ser Leu Pro Ser Arg Arg Met Gln Asn Asp Thr Ala Glu Asn
       130                 135                 140
   Glu Thr Thr Glu Lys Glu Glu Lys Ser Glu Ser Arg Gln Glu Arg Tyr
   145                 150                 155                 160
   Glu Ile Glu Glu Thr Glu Thr Val Thr Lys Ser Tyr Gln Lys Asn Asp
                   165                 170                 175
   Trp Arg Asp Ala Glu Glu Asn Lys Lys Glu Asp Lys Glu Lys Glu Glu
               180                 185                 190
   Glu Glu Glu Glu Lys Pro Lys Arg Gly Ser Ile Gly Glu Asn Gln Val
           195                 200                 205
   Glu Val Met Val Glu Glu Lys Thr Thr Glu Ser Gln Glu Glu Thr Val
       210                 215                 220
   Val Met Ser Leu Lys Asn Gly Gln Ile Ser Ser Glu Glu Pro Lys Gln
   225                 230                 235                 240
   Glu Glu Glu Arg Glu Gln Gly Ser Asp Glu Ile Ser His His Glu Lys
                   245                 250                 255
   Met Glu Glu Glu Asp Lys Glu Arg Ala Glu Ala Glu Arg Ala Arg Leu
               260                 265                 270
   Glu Ala Glu Glu Arg Glu Arg Ile Lys Ala Glu Gln Asp Lys Lys Ile
           275                 280                 285
   Ala Asp Glu Arg Ala Arg Ile Glu Ala Glu Glu Lys Ala Ala Ala Gln
       290                 295                 300
   Glu Arg Glu Arg Arg Glu Ala Glu Glu Arg Glu Arg Met Arg Glu Glu
   305                 310                 315                 320
   Glu Lys Arg Ala Ala Glu Glu Arg Gln Arg Ile Lys Glu Glu Glu Lys
                   325                 330                 335
   Arg Ala Ala Glu Glu Arg Gln Arg Ile Lys Glu Glu Glu Lys Arg Ala
               340                 345                 350
   Ala Glu Glu Arg Gln Arg Ile Lys Glu Glu Glu Lys Arg Ala Ala Glu
           355                 360                 365
   Glu Arg Gln Arg Ala Arg Ala Glu Glu Glu Glu Lys Ala Lys Val Glu
       370                 375                 380
   Glu Gln Lys Arg Asn Lys Gln Leu Glu Glu Lys Lys Arg Ala Met Gln
   385                 390                 395                 400
   Glu Thr Lys Ile Lys Gly Glu Lys Val Glu Gln Lys Ile Glu Gly Lys
                   405                 410                 415
   Trp Val Asn Glu Lys Lys Ala Gln Glu Asp Lys Leu Gln Thr Ala Val
               420                 425                 430
   Leu Lys Lys Gln Gly Glu Glu Lys Gly Thr Lys Val Gln Ala Lys Arg
               435                 440                 445
   Glu Lys Leu Gln Glu Asp Lys Pro Thr Phe Lys Lys Glu Glu Ile Lys
       450                 455                 460
   Asp Glu Lys Ile Lys Lys Asp Lys Glu Pro Lys Glu Glu Val Lys Ser
   465                 470                 475                 480
   Phe Met Asp Arg Lys Lys Gly Phe Thr Glu Val Lys Ser Gln Asn Gly
                   485                 490                 495
   Glu Phe Met Thr His Lys Leu Lys His Thr Glu Asn Thr Phe Ser Arg
               500                 505                 510
   Pro Gly Gly Arg Ala Ser Val Asp Thr Lys Glu Ala Glu Gly Ala Pro
           515                 520                 525
   Gln Val Glu Ala Gly Lys Arg Leu Glu Glu Leu Arg Arg Arg Arg Gly
       530                 535                 540
   Glu Thr Glu Ser Glu Glu Phe Glu Lys Leu Lys Gln Lys Gln Gln Glu
   545                 550                 555                 560
   Ala Ala Leu Glu Leu Glu Glu Leu Lys Lys Lys Arg Glu Glu Arg Arg
                   565                 570                 575
   Lys Val Leu Glu Glu Glu Glu Gln Arg Arg Lys Gln Glu Glu Ala Asp
               580                 585                 590
   Arg Lys Leu Arg Glu Glu Glu Glu Lys Arg Arg Leu Lys Glu Glu Ile
           595                 600                 605
   Glu Arg Arg Arg Ala Glu Ala Ala Glu Lys Arg Gln Lys Met Pro Glu
       610                 615                 620
   Asp Gly Leu Ser Asp Asp Lys Lys Pro Phe Lys Cys Phe Thr Pro Lys
   625                 630                 635                 640
   Gly Ser Ser Leu Lys Ile Glu Glu Arg Ala Glu Phe Leu Asn Lys Ser
                   645                 650                 655
   Val Gln Lys Ser Ser Gly Val Lys Ser Thr His Gln Ala Ala Ile Val
               660                 665                 670
   Ser Lys Ile Asp Ser Arg Leu Glu Gln Tyr Thr Ser Ala Ile Glu Gly
           675                 680                 685
   Thr Lys Ser Ala Lys Pro Thr Lys Pro Ala Ala Ser Asp Leu Pro Val
       690                 695                 700
   Pro Ala Glu Gly Val Arg Asn Ile Lys Ser Met Trp Glu Lys Gly Asn
   705                 710                 715                 720
   Val Phe Ser Ser Pro Thr Ala Ala Gly Thr Pro Asn Lys Glu Thr Ala
                   725                 730                 735
   Gly Leu Lys Val Gly Val Ser Ser Arg Ile Asn Glu Trp Leu Thr Lys
               740                 745                 750
   Thr Pro Asp Gly Asn Lys Ser Pro Ala Pro Lys Pro Ser Asp Leu Arg
           755                 760                 765
   Pro Gly Asp Val Ser Ser Lys Arg Asn Leu Trp Glu Lys Gln Ser Val
       770                 775                 780
   Asp Lys Val Thr Ser Pro Thr Lys Val
   785                 790
<210> 6
   <211> 240
   <212> PRT
   <213> Homo sapiens
<400> 6
   Met Asp Pro Arg Lys Val Asn Glu Leu Arg Ala Phe Val Lys Met Cys
   1               5                   10                  15
   Lys Lys Asp Pro Ser Ile Leu His Thr Gln Glu Met Arg Phe Leu Arg
               20                  25                  30
   Glu Trp Val Glu Ser Met Gly Gly Thr Ala Thr Gln Lys Ala Lys Ser
           35                  40                  45
   Glu Glu Asn Thr Lys Glu Glu Lys Pro Asp Ser Lys Val Glu Glu Asp
       50                  55                  60
   Leu Lys Ala Asp Glu Pro Ser Ser Glu Glu Ser Asp Leu Glu Ile Asp
   65                  70                  75                  80
   Lys Glu Gly Val Ile Glu Pro Asp Thr Asp Ala Pro Gln Glu Met Gly
                   85                  90                  95
   Asp Glu Asn Ala Glu Ile Thr Glu Glu Val Met Asp Gln Ala Asn Asp
               100                 105                 110
   Lys Lys Val Ala Ala Ile Glu Ala Leu Asn Asp Gly Glu Leu Gln Lys
           115                 120                 125
   Ala Ile Asp Leu Phe Thr Asp Ala Ile Lys Leu Asn Pro Arg Leu Ala
       130                 135                 140
   Ile Leu Tyr Ala Lys Arg Ala Ser Val Phe Val Lys Leu Gln Lys Pro
   145                 150                 155                 160
   Asn Ala Ala Ile Arg Asp Cys Asp Arg Ala Ile Glu Ile Asn Pro Asp
                   165                 170                 175
   Ser Ala Gln Pro Tyr Lys Arg Arg Gly Lys Ala His Arg Leu Leu Gly
               180                 185                 190
   His Trp Glu Glu Ala Ala His Asp Leu Ala Leu Ala Cys Lys Phe Asp
           195                 200                 205
   Tyr Asp Glu Asp Ala Ser Ala Met Leu Lys Glu Val Gln Pro Arg Ala
       210                 215                 220
   Gln Lys Ile Ala Glu His Gln Arg Lys Tyr Glu Arg Lys Arg Glu Glu
   225                 230                 235                 240
<210> 7
   <211> 710
   <212> PRT
   <213> Homo sapiens
<400> 7
   Met Ser Asp Tyr Ser Thr Gly Gly Pro Pro Pro Gly Pro Pro Pro Pro
   1               5                   10                  15
   Ala Gly Gly Gly Gly Gly Ala Gly Gly Ala Gly Gly Gly Pro Pro Pro
               20                  25                  30
   Gly Pro Pro Gly Ala Gly Asp Arg Gly Gly Gly Gly Pro Cys Gly Gly
           35                  40                  45
   Gly Pro Gly Gly Gly Ser Ala Gly Gly Pro Ser Gln Pro Pro Gly Gly
       50                  55                  60
   Gly Gly Pro Gly Ile Arg Lys Asp Ala Phe Ala Asp Ala Val Gln Arg
   65                  70                  75                  80
   Ala Arg Gln Ile Ala Ala Lys Ile Gly Gly Asp Ala Ala Thr Thr Val
                   85                  90                  95
   Asn Asn Ser Thr Pro Asp Phe Gly Phe Gly Gly Gln Lys Arg Gln Leu
               100                 105                 110
   Glu Asp Gly Asp Gln Pro Glu Ser Lys Lys Leu Ala Ser Gln Gly Asp
           115                 120                 125
   Ser Ile Ser Ser Gln Leu Gly Pro Ile His Pro Pro Pro Arg Thr Ser
       130                 135                 140
   Met Thr Glu Glu Tyr Arg Val Pro Asp Gly Met Val Gly Leu Ile Ile
   145                 150                 155                 160
   Gly Arg Gly Gly Glu Gln Ile Asn Lys Ile Gln Gln Asp Ser Gly Cys
                   165                 170                 175
   Lys Val Gln Ile Ser Pro Asp Ser Gly Gly Leu Pro Glu Arg Ser Val
               180                 185                 190
   Ser Leu Thr Gly Ala Pro Glu Ser Val Gln Lys Ala Lys Met Met Leu
           195                 200                 205
   Asp Asp Ile Val Ser Arg Gly Arg Gly Gly Pro Pro Gly Gln Phe His
       210                 215                 220
   Asp Asn Ala Asn Gly Gly Gln Asn Gly Thr Val Gln Glu Ile Met Ile
   225                 230                 235                 240
   Pro Ala Gly Lys Ala Gly Leu Val Ile Gly Lys Gly Gly Glu Thr Ile
                   245                 250                 255
   Lys Gln Leu Gln Glu Arg Ala Gly Val Lys Met Ile Leu Ile Gln Asp
               260                 265                 270
   Gly Ser Gln Asn Thr Asn Val Asp Lys Pro Leu Arg Ile Ile Gly Asp
           275                 280                 285
   Pro Tyr Lys Val Gln Gln Ala Cys Glu Met Val Met Asp Ile Leu Arg
       290                 295                 300
   Glu Arg Asp Gln Gly Gly Phe Gly Asp Arg Asn Glu Tyr Gly Ser Arg
   305                 310                 315                 320
   Ile Gly Gly Gly Ile Asp Val Pro Val Pro Arg His Ser Val Gly Val
                   325                 330                 335
   Val Ile Gly Arg Ser Gly Glu Met Ile Lys Lys Ile Gln Asn Asp Ala
               340                 345                 350
   Gly Val Arg Ile Gln Phe Lys Gln Asp Asp Gly Thr Gly Pro Glu Lys
           355                 360                 365
   Ile Ala His Ile Met Gly Pro Pro Asp Arg Cys Glu His Ala Ala Arg
       370                 375                 380
   Ile Ile Asn Asp Leu Leu Gln Ser Leu Arg Ser Gly Pro Pro Gly Pro
   385                 390                 395                 400
   Pro Gly Gly Pro Gly Met Pro Pro Gly Gly Arg Gly Arg Gly Arg Gly
                   405                 410                 415
   Gln Gly Asn Trp Gly Pro Pro Gly Gly Glu Met Thr Phe Ser Ile Pro
               420                 425                 430
   Thr His Lys Cys Gly Leu Val Ile Gly Arg Gly Gly Glu Asn Val Lys
           435                 440                 445
   Ala Ile Asn Gln Gln Thr Gly Ala Phe Val Glu Ile Ser Arg Gln Leu
       450                 455                 460
   Pro Pro Asn Gly Asp Pro Asn Phe Lys Leu Phe Ile Ile Arg Gly Ser
   465                 470                 475                 480
   Pro Gln Gln Ile Asp His Ala Lys Gln Leu Ile Glu Glu Lys Ile Glu
                   485                 490                 495
   Gly Pro Leu Cys Pro Val Gly Pro Gly Pro Gly Gly Pro Gly Pro Ala
               500                 505                 510
   Gly Pro Met Gly Pro Phe Asn Pro Gly Pro Phe Asn Gln Gly Pro Pro
           515                 520                 525
   Gly Ala Pro Pro His Ala Gly Gly Pro Pro Pro His Gln Tyr Pro Pro
       530                 535                 540
   Gln Gly Trp Gly Asn Thr Tyr Pro Gln Trp Gln Pro Pro Ala Pro His
   545                 550                 555                 560
   Asp Pro Ser Lys Ala Ala Ala Ala Ala Ala Asp Pro Asn Ala Ala Trp
                   565                 570                 575
   Ala Ala Tyr Tyr Ser His Tyr Tyr Gln Gln Pro Pro Gly Pro Val Pro
               580                 585                 590
   Gly Pro Ala Pro Ala Pro Ala Ala Pro Pro Ala Gln Gly Glu Pro Pro
           595                 600                 605
   Gln Pro Pro Pro Thr Gly Gln Ser Asp Tyr Thr Lys Ala Trp Glu Glu
       610                 615                 620
   Tyr Tyr Lys Lys Ile Gly Gln Gln Pro Gln Gln Pro Gly Ala Pro Pro
   625                 630                 635                 640
   Gln Gln Asp Tyr Thr Lys Ala Trp Glu Glu Tyr Tyr Lys Lys Gln Ala
                   645                 650                 655
   Gln Val Ala Thr Gly Gly Gly Pro Gly Ala Pro Pro Gly Ser Gln Pro
               660                 665                 670
   Asp Tyr Ser Ala Ala Trp Ala Glu Tyr Tyr Arg Gln Gln Ala Ala Tyr
           675                 680                 685
   Tyr Gly Gln Thr Pro Val Pro Gly Pro Gln Pro Pro Pro Thr Gln Gln
       690                 695                 700
   Gly Gln Gln Gln Ala Gln
   705                 710
<210> 8
   <211> 375
   <212> PRT
   <213> Homo sapiens
<400> 8
<210> 9
   <211> 434
   <212> PRT
   <213> Homo sapiens
<400> 9
   Met Ser Ile Glu Lys Ile Trp Ala Arg Glu Ile Leu Asp Ser Arg Gly
   1               5                   10                  15
   Asn Pro Thr Val Glu Val Asp Leu Tyr Thr Ala Lys Gly Leu Phe Arg
               20                  25                  30
   Ala Ala Val Pro Ser Gly Ala Ser Thr Gly Ile Tyr Glu Ala Leu Glu
           35                  40                  45
   Leu Arg Asp Gly Asp Lys Gln Arg Tyr Leu Gly Lys Gly Val Leu Lys
       50                  55                  60
   Ala Val Asp His Ile Asn Ser Thr Ile Ala Pro Ala Leu Ile Ser Ser
   65                  70                  75                  80
   Gly Leu Ser Val Val Glu Gln Glu Lys Leu Asp Asn Leu Met Leu Glu
                   85                  90                  95
   Leu Asp Gly Thr Glu Asn Lys Ser Lys Phe Gly Ala Asn Ala Ile Leu
               100                 105                 110
   Gly Val Ser Leu Ala Val Cys Lys Ala Gly Ala Ala Glu Arg Glu Leu
           115                 120                 125
   Pro Leu Tyr Arg His Ile Ala Gln Leu Ala Gly Asn Ser Asp Leu Ile
       130                 135                 140
   Leu Pro Val Pro Ala Phe Asn Val Ile Asn Gly Gly Ser His Ala Gly
   145                 150                 155                 160
   Asn Lys Leu Ala Met Gln Glu Phe Met Ile Leu Pro Val Gly Ala Glu
                   165                 170                 175
   Ser Phe Arg Asp Ala Met Arg Leu Gly Ala Glu Val Tyr His Thr Leu
               180                 185                 190
   Lys Gly Val Ile Lys Asp Lys Tyr Gly Lys Asp Ala Thr Asn Val Gly
           195                 200                 205
   Asp Glu Gly Gly Phe Ala Pro Asn Ile Leu Glu Asn Ser Glu Ala Leu
       210                 215                 220
   Glu Leu Val Lys Glu Ala Ile Asp Lys Ala Gly Tyr Thr Glu Lys Ile
   225                 230                 235                 240
   Val Ile Gly Met Asp Val Ala Ala Ser Glu Phe Tyr Arg Asp Gly Lys
                   245                 250                 255
   Tyr Asp Leu Asp Phe Lys Ser Pro Thr Asp Pro Ser Arg Tyr Ile Thr
               260                 265                 270
   Gly Asp Gln Leu Gly Ala Leu Tyr Gln Asp Phe Val Arg Asp Tyr Pro
           275                 280                 285
   Val Val Ser Ile Glu Asp Pro Phe Asp Gln Asp Asp Trp Ala Ala Trp
       290                 295                 300
   Ser Lys Phe Thr Ala Asn Val Gly Ile Gln Ile Val Gly Asp Asp Leu
   305                 310                 315                 320
   Thr Val Thr Asn Pro Lys Arg Ile Glu Arg Ala Val Glu Glu Lys Ala
                   325                 330                 335
   Cys Asn Cys Leu Leu Leu Lys Val Asn Gln Ile Gly Ser Val Thr Glu
               340                 345                 350
   Ala Ile Gln Ala Cys Lys Leu Ala Gln Glu Asn Gly Trp Gly Val Met
           355                 360                 365
   Val Ser His Arg Ser Gly Glu Thr Glu Asp Thr Phe Ile Ala Asp Leu
   370 375 380
   Val Val Gly Leu Cys Thr Gly Gln Ile Lys Thr Gly Ala Pro Cys Arg
   385 390 395 400
   Ser Glu Arg Leu Ala Lys Tyr Asn Gln Leu Met Arg Ile Glu Glu Glu
   405 410 415
   Leu Gly Asp Glu Ala Arg Phe Ala Gly His Asn Phe Arg Asn Pro Ser
   420 425 430
   Val Leu
<210> 10
   <211> 505
   <212> PRT
   <213> Homo sapiens
<400> 10
   Met Arg Leu Arg Arg Leu Ala Leu Phe Pro Gly Val Ala Leu Leu Leu
   1 5 10 15
   Ala Ala Ala Arg Leu Ala Ala Ala Ser Asp Val Leu Glu Leu Thr Asp
   20 25 30
   Asp Asn Phe Glu Ser Arg Ile Ser Asp Thr Gly Ser Ala Gly Leu Met
   35 40 45
   Leu Val Glu Phe Phe Ala Pro Trp Cys Gly His Cys Lys Arg Leu Ala
   50 55 60
   Pro Glu Tyr Glu Ala Ala Ala Thr Arg Leu Lys Gly Ile Val Pro Leu
   65 70 75 80
   Ala Lys Val Asp Cys Thr Ala Asn Thr Asn Thr Cys Asn Lys Tyr Gly
   85 90 95
   Val Ser Gly Tyr Pro Thr Leu Lys Ile Phe Arg Asp Gly Glu Glu Ala
   100 105 110
   Gly Ala Tyr Asp Gly Pro Arg Thr Ala Asp Gly Ile Val Ser His Leu
   115 120 125
   Lys Lys Gln Ala Gly Pro Ala Ser Val Pro Leu Arg Thr Glu Glu Glu
   130 135 140
   Phe Lys Lys Phe Ile Ser Asp Lys Asp Ala Ser Ile Val Gly Phe Phe
   145                 150                 155                 160
   Asp Asp Ser Phe Ser Glu Ala His Ser Glu Phe Leu Lys Ala Ala Ser
                   165                 170                 175
   Asn Leu Arg Asp Asn Tyr Arg Phe Ala His Thr Asn Val Glu Ser Leu
               180                 185                 190
   Val Asn Glu Tyr Asp Asp Asn Gly Glu Gly Ile Ile Leu Phe Arg Pro
           195                 200                 205
   Ser His Leu Thr Asn Lys Phe Glu Asp Lys Thr Val Ala Tyr Thr Glu
       210                 215                 220
   Gln Lys Met Thr Ser Gly Lys Ile Lys Lys Phe Ile Gln Glu Asn Ile
   225                 230                 235                 240
   Phe Gly Ile Cys Pro His Met Thr Glu Asp Asn Lys Asp Leu Ile Gln
                   245                 250                 255
   Gly Lys Asp Leu Leu Ile Ala Tyr Tyr Asp Val Asp Tyr Glu Lys Asn
               260                 265                 270
   Ala Lys Gly Ser Asn Tyr Trp Arg Asn Arg Val Met Met Val Ala Lys
           275                 280                 285
   Lys Phe Leu Asp Ala Gly His Lys Leu Asn Phe Ala Val Ala Ser Arg
       290                 295                 300
   Lys Thr Phe Ser His Glu Leu Ser Asp Phe Gly Leu Glu Ser Thr Ala
   305                 310                 315                 320
   Gly Glu Ile Pro Val Val Ala Ile Arg Thr Ala Lys Gly Glu Lys Phe
                   325                 330                 335
   Val Met Gln Glu Glu Phe Ser Arg Asp Gly Lys Ala Leu Glu Arg Phe
               340                 345                 350
   Leu Gln Asp Tyr Phe Asp Gly Asn Leu Lys Arg Tyr Leu Lys Ser Glu
           355                 360                 365
   Pro Ile Pro Glu Ser Asn Asp Gly Pro Val Lys Val Val Val Ala Glu
       370                 375                 380
   Asn Phe Asp Glu Ile Val Asn Asn Glu Asn Lys Asp Val Leu Ile Glu
   385                 390                 395                 400
   Phe Tyr Ala Pro Trp Cys Gly His Cys Lys Asn Leu Glu Pro Lys Tyr
                   405                 410                 415
   Lys Glu Leu Gly Glu Lys Leu Ser Lys Asp Pro Asn Ile Val Ile Ala
               420                 425                 430
   Lys Met Asp Ala Thr Ala Asn Asp Val Pro Ser Pro Tyr Glu Val Arg
           435                 440                 445
   Gly Phe Pro Thr Ile Tyr Phe Ser Pro Ala Asn Lys Lys Leu Asn Pro
       450                 455                 460
   Lys Lys Tyr Glu Gly Gly Arg Glu Leu Ser Asp Phe Ile Ser Tyr Leu
   465                 470                 475                 480
   Gln Arg Glu Ala Thr Asn Pro Pro Val Ile Gln Glu Glu Lys Pro Lys
                   485                 490                 495
   Lys Lys Lys Lys Ala Gln Glu Asp Leu
               500                 505
<210> 11
   <211> 470
   <212> PRT
   <213> Homo sapiens
<400> 11
<210> 12
   <211> 470
   <212> PRT
   <213> Homo sapiens
<400> 12
   Met Ser His His Pro Ser Gly Leu Arg Ala Gly Phe Ser Ser Thr Ser
   1               5                   10                  15
   Tyr Arg Arg Thr Phe Gly Pro Pro Pro Ser Leu Ser Pro Gly Ala Phe
               20                  25                  30
   Ser Tyr Ser Ser Ser Ser Arg Phe Ser Ser Ser Arg Leu Leu Gly Ser
           35                  40                  45
   Ala Ser Pro Ser Ser Ser Val Arg Leu Gly Ser Phe Arg Ser Pro Arg
       50                  55                  60
   Ala Gly Ala Gly Ala Leu Leu Arg Leu Pro Ser Glu Arg Leu Asp Phe
   65                  70                  75                  80
   Ser Met Ala Glu Ala Leu Asn Gln Glu Phe Leu Ala Thr Arg Ser Asn
                   85                  90                  95
   Glu Lys Gln Glu Leu Gln Glu Leu Asn Asp Arg Phe Ala Asn Phe Ile
               100                 105                 110
   Glu Lys Val Arg Phe Leu Glu Gln Gln Asn Ala Ala Leu Arg Gly Glu
           115                 120                 125
   Leu Ser Gln Ala Arg Gly Gln Glu Pro Ala Arg Ala Asp Gln Leu Cys
       130                 135                 140
   Gln Gln Glu Leu Arg Glu Leu Arg Arg Glu Leu Glu Leu Leu Gly Arg
   145                 150                 155                 160
   Glu Arg Asp Arg Val Gln Val Glu Arg Asp Gly Leu Ala Glu Asp Leu
                   165                 170                 175
   Ala Ala Leu Lys Gln Arg Leu Glu Glu Glu Thr Arg Lys Arg Glu Asp
               180                 185                 190
   Ala Glu His Asn Leu Val Leu Phe Arg Lys Asp Val Asp Asp Ala Thr
           195                 200                 205
   Leu Ser Arg Leu Glu Leu Glu Arg Lys Ile Glu Ser Leu Met Asp Glu
       210                 215                 220
   Ile Glu Phe Leu Lys Lys Leu His Glu Glu Glu Leu Arg Asp Leu Gln
   225                 230                 235                 240
   Val Ser Val Glu Ser Gln Gln Val Gln Gln Val Glu Val Glu Ala Thr
                   245                 250                 255
   Val Lys Pro Glu Leu Thr Ala Ala Leu Arg Asp Ile Arg Ala Gln Tyr
               260                 265                 270
   Glu Ser Ile Ala Ala Lys Asn Leu Gln Glu Ala Glu Glu Trp Tyr Lys
           275                 280                 285
   Ser Lys Tyr Ala Asp Leu Ser Asp Ala Ala Asn Arg Asn His Glu Ala
       290                 295                 300
   Leu Arg Gln Ala Lys Gln Glu Met Asn Glu Ser Arg Arg Gln Ile Gln
   305 310 315 320
   Ser Leu Thr Cys Glu Val Asp Gly Leu Arg Gly Thr Asn Glu Ala Leu
   325 330 335
   Leu Arg Gln Leu Arg Glu Leu Glu Glu Gln Phe Ala Leu Glu Ala Gly
   340 345 350
   Gly Tyr Gln Ala Gly Ala Ala Arg Leu Glu Glu Glu Leu Arg Gln Leu
   355 360 365
   Lys Glu Glu Met Ala Arg His Leu Arg Glu Tyr Gln Glu Leu Leu Asn
   370 375 380
   Val Lys Met Ala Leu Asp Ile Glu Ile Ala Thr Tyr Arg Lys Leu Leu
   385 390 395 400
   Glu Gly Glu Glu Ser Arg Ile Ser Val Pro Val His Ser Phe Ala Ser
   405 410 415
   Leu Asn Ile Lys Thr Thr Val Pro Glu Val Glu Pro Pro Gln Asp Ser
   420 425 430
   His Ser Arg Lys Thr Val Leu Ile Lys Thr Ile Glu Thr Arg Asn Gly
   435 440 445
   Glu Val Val Thr Glu Ser Gln Lys Glu Gln Arg Ser Glu Leu Asp Lys
   450 455 460
   Ser Ser Ala His Ser Tyr
   465 470
<210> 13
   <211> 449
   <212> PRT
   <213> Homo sapiens
<400> 13
   Met Met Leu Gly Thr Glu Gly Gly Glu Gly Phe Val Val Lys Val Arg
   1 5 10 15
   Gly Leu Pro Trp Ser Cys Ser Ala Asp Glu Val Gln Arg Phe Phe Ser
   20 25 30
   Asp Cys Lys Ile Gln Asn Gly Ala Gln Gly Ile Arg Phe Ile Tyr Thr
   35 40 45
   Arg Glu Gly Arg Pro Ser Gly Glu Ala Phe Val Glu Leu Glu Ser Glu
       50                  55                  60
   Asp Glu Val Lys Leu Ala Leu Lys Lys Asp Arg Glu Thr Met Gly His
   65                  70                  75                  80
   Arg Tyr Val Glu Val Phe Lys Ser Asn Asn Val Glu Met Asp Trp Val
                   85                  90                  95
   Leu Lys His Thr Gly Pro Asn Ser Pro Asp Thr Ala Asn Asp Gly Phe
               100                 105                 110
   Val Arg Leu Arg Gly Leu Pro Phe Gly Cys Ser Lys Glu Glu Ile Val
           115                 120                 125
   Gln Phe Phe Ser Gly Leu Glu Ile Val Pro Asn Gly Ile Thr Leu Pro
       130                 135                 140
   Val Asp Phe Gln Gly Arg Ser Thr Gly Glu Ala Phe Val Gln Phe Ala
   145                 150                 155                 160
   Ser Gln Glu Ile Ala Glu Lys Ala Leu Lys Lys His Lys Glu Arg Ile
                   165                 170                 175
   Gly His Arg Tyr Ile Glu Ile Phe Lys Ser Ser Arg Ala Glu Val Arg
               180                 185                 190
   Thr His Tyr Asp Pro Pro Arg Lys Leu Met Ala Met Gln Arg Pro Gly
           195                 200                 205
   Pro Tyr Asp Arg Pro Gly Ala Gly Arg Gly Tyr Asn Ser Ile Gly Arg
       210                 215                 220
   Gly Ala Gly Phe Glu Arg Met Arg Arg Gly Ala Tyr Gly Gly Gly Tyr
   225                 230                 235                 240
   Gly Gly Tyr Asp Asp Tyr Asn Gly Tyr Asn Asp Gly Tyr Gly Phe Gly
                   245                 250                 255
   Ser Asp Arg Phe Gly Arg Asp Leu Asn Tyr Cys Phe Ser Gly Met Ser
               260                 265                 270
   Asp His Arg Tyr Gly Asp Gly Gly Ser Thr Phe Gln Ser Thr Thr Gly
           275                 280                 285
   His Cys Val His Met Arg Gly Leu Pro Tyr Arg Ala Thr Glu Asn Asp
   290 295 300
   Ile Tyr Asn Phe Phe Ser Pro Leu Asn Pro Val Arg Val His Ile Glu
   305 310 315 320
   Ile Gly Pro Asp Gly Arg Val Thr Gly Glu Ala Asp Val Glu Phe Ala
   325 330 335
   Thr His Glu Asp Ala Val Ala Ala Met Ser Lys Asp Lys Ala Asn Met
   340 345 350
   Gln His Arg Tyr Val Glu Leu Phe Leu Asn Ser Thr Ala Gly Ala Ser
   355 360 365
   Gly Gly Ala Tyr Glu His Arg Tyr Val Glu Leu Phe Leu Asn Ser Thr
   370 375 380
   Ala Gly Ala Ser Gly Gly Ala Tyr Gly Ser Gln Met Met Gly Gly Met
   385 390 395 400
   Gly Leu Ser Asn Gln Ser Ser Tyr Gly Gly Pro Ala Ser Gln Gln Leu
   405 410 415
   Ser Gly Gly Tyr Gly Gly Gly Tyr Gly Gly Gln Ser Ser Met Ser Gly
   420 425 430
   Tyr Asp Gln Val Leu Gln Glu Asn Ser Ser Asp Phe Gln Ser Asn Ile
   435 440 445
   Ala
<210> 14
   <211> 543
   <212> PRT
   <213> Homo sapiens
<400> 14
   Met Glu Gln Val Asn Glu Leu Lys Glu Lys Gly Asn Lys Ala Leu Ser
   1 5 10 15
   Val Gly Asn Ile Asp Asp Ala Leu Gln Cys Tyr Ser Glu Ala Ile Lys
   20 25 30
   Leu Asp Pro His Asn His Val Leu Tyr Ser Asn Arg Ser Ala Ala Tyr
   35 40 45
   Ala Lys Lys Gly Asp Tyr Gln Lys Ala Tyr Glu Asp Gly Cys Lys Thr
       50                  55                  60
   Val Asp Leu Lys Pro Asp Trp Gly Lys Gly Tyr Ser Arg Lys Ala Ala
   65                  70                  75                  80
   Ala Leu Glu Phe Leu Asn Arg Phe Glu Glu Ala Lys Arg Thr Tyr Glu
                   85                  90                  95
   Glu Gly Leu Lys His Glu Ala Asn Asn Pro Gln Leu Lys Glu Gly Leu
               100                 105                 110
   Gln Asn Met Glu Ala Arg Leu Ala Glu Arg Lys Phe Met Asn Pro Phe
           115                 120                 125
   Asn Met Pro Asn Leu Tyr Gln Lys Leu Glu Ser Asp Pro Arg Thr Arg
       130                 135                 140
   Thr Leu Leu Ser Asp Pro Thr Tyr Arg Glu Leu Ile Glu Gln Leu Arg
   145                 150                 155                 160
   Asn Lys Pro Ser Asp Leu Gly Thr Lys Leu Gln Asp Pro Arg Ile Met
                   165                 170                 175
   Thr Thr Leu Ser Val Leu Leu Gly Val Asp Leu Gly Ser Met Asp Glu
               180                 185                 190
   Glu Glu Glu Ile Ala Thr Pro Pro Pro Pro Pro Pro Pro Lys Lys Glu
           195                 200                 205
   Thr Lys Pro Glu Pro Met Glu Glu Asp Leu Pro Glu Asn Lys Lys Gln
       210                 215                 220
   Ala Leu Lys Glu Lys Glu Leu Gly Asn Asp Ala Tyr Lys Lys Lys Asp
   225                 230                 235                 240
   Phe Asp Thr Ala Leu Lys His Tyr Asp Lys Ala Lys Glu Leu Asp Pro
                   245                 250                 255
   Thr Asn Met Thr Tyr Ile Thr Asn Gln Ala Ala Val Tyr Phe Glu Lys
               260                 265                 270
   Gly Asp Tyr Asn Lys Cys Arg Glu Leu Cys Glu Lys Ala Ile Glu Val
           275                 280                 285
   Gly Arg Glu Asn Arg Glu Asp Tyr Arg Gln Ile Ala Lys Ala Tyr Ala
       290                 295                 300
   Arg Ile Gly Asn Ser Tyr Phe Lys Glu Glu Lys Tyr Lys Asp Ala Ile
   305                 310                 315                 320
   His Phe Tyr Asn Lys Ser Leu Ala Glu His Arg Thr Pro Asp Val Leu
                   325                 330                 335
   Lys Lys Cys Gln Gln Ala Glu Lys Ile Leu Lys Glu Gln Glu Arg Leu
               340                 345                 350
   Ala Tyr Ile Asn Pro Asp Leu Ala Leu Glu Glu Lys Asn Lys Gly Asn
           355                 360                 365
   Glu Cys Phe Gln Lys Gly Asp Tyr Pro Gln Ala Met Lys His Tyr Thr
       370                 375                 380
   Glu Ala Ile Lys Arg Asn Pro Lys Asp Ala Lys Leu Tyr Ser Asn Arg
   385                 390                 395                 400
   Ala Ala Cys Tyr Thr Lys Leu Leu Glu Phe Gln Leu Ala Leu Lys Asp
                   405                 410                 415
   Cys Glu Glu Cys Ile Gln Leu Glu Pro Thr Phe Ile Lys Gly Tyr Thr
               420                 425                 430
   Arg Lys Ala Ala Ala Leu Glu Ala Met Lys Asp Tyr Thr Lys Ala Met
           435                 440                 445
   Asp Val Tyr Gln Lys Ala Leu Asp Leu Asp Ser Ser Cys Lys Glu Ala
       450                 455                 460
   Ala Asp Gly Tyr Gln Arg Cys Met Met Ala Gln Tyr Asn Arg His Asp
   465                 470                 475                 480
   Ser Pro Glu Asp Val Lys Arg Arg Ala Met Ala Asp Pro Glu Val Gln
                   485                 490                 495
   Gln Ile Met Ser Asp Pro Ala Met Arg Leu Ile Leu Glu Gln Met Gln
               500                 505                 510
   Lys Asp Pro Gln Ala Leu Ser Glu His Leu Lys Asn Pro Val Ile Ala
           515                 520                 525
   Gln Lys Ile Gln Lys Leu Met Asp Val Gly Leu Ile Ala Ile Arg
   530 535 540
<210> 15
   <211> 249
   <212> PRT
   <213> Homo sapiens
<400> 15
   Met Ala Pro Ser Arg Lys Phe Phe Val Gly Gly Asn Trp Lys Met Asn
   1               5                   10                  15
   Gly Arg Lys Gln Ser Leu Gly Glu Leu Ile Gly Thr Leu Asn Ala Ala
               20                  25                  30
   Lys Val Pro Ala Asp Thr Glu Val Val Cys Ala Pro Pro Thr Ala Tyr
           35                  40                  45
   Ile Asp Phe Ala Arg Gln Lys Leu Asp Pro Lys Ile Ala Val Ala Ala
       50                  55                  60
   Gln Asn Cys Tyr Lys Val Thr Asn Gly Ala Phe Thr Gly Glu Ile Ser
   65                  70                  75                  80
   Pro Gly Met Ile Lys Asp Cys Gly Ala Thr Trp Val Val Leu Gly His
                   85                  90                  95
   Ser Glu Arg Arg His Val Phe Gly Glu Ser Asp Glu Leu Ile Gly Gln
               100                 105                 110
   Lys Val Ala His Ala Leu Ala Glu Gly Leu Gly Val Ile Ala Cys Ile
           115                 120                 125
   Gly Glu Lys Leu Asp Glu Arg Glu Ala Gly Ile Thr Glu Lys Val Val
       130                 135                 140
   Phe Glu Gln Thr Lys Val Ile Ala Asp Asn Val Lys Asp Trp Ser Lys
   145                 150                 155                 160
   Val Val Leu Ala Tyr Glu Pro Val Trp Ala Ile Gly Thr Gly Lys Thr
                   165                 170                 175
   Ala Thr Pro Gln Gln Ala Gln Glu Val His Glu Lys Leu Arg Gly Trp
               180                 185                 190
   Leu Lys Ser Asn Val Ser Asp Ala Val Ala Gln Ser Thr Arg Ile Ile
           195                 200                 205
   Tyr Gly Gly Ser Val Thr Gly Ala Thr Cys Lys Glu Leu Ala Ser Gln
       210                 215                 220
   Pro Asp Val Asp Gly Phe Leu Val Gly Gly Ala Ser Leu Lys Pro Glu
   225                 230                 235                 240
   Phe Val Asp Ile Ile Asn Ala Lys Gln
                   245
<210> 16
   <211> 224
   <212> PRT
   <213> Homo sapiens
<400> 16
   Met Pro Gly Gly Leu Leu Leu Gly Asp Val Ala Pro Asn Phe Glu Ala
   1               5                   10                  15
   Asn Thr Thr Val Gly Arg Ile Arg Phe His Asp Phe Leu Gly Asp Ser
               20                  25                  30
   Trp Gly Ile Leu Phe Ser His Pro Arg Asp Phe Thr Pro Val Cys Thr
           35                  40                  45
   Thr Glu Leu Gly Arg Ala Ala Lys Leu Ala Pro Glu Phe Ala Lys Arg
       50                  55                  60
   Asn Val Lys Leu Ile Ala Leu Ser Ile Asp Ser Val Glu Asp His Leu
   65                  70                  75                  80
   Ala Trp Ser Lys Asp Ile Asn Ala Tyr Asn Cys Glu Glu Pro Thr Glu
                   85                  90                  95
   Lys Leu Pro Phe Pro Ile Ile Asp Asp Arg Asn Arg Glu Leu Ala Ile
               100                 105                 110
   Leu Leu Gly Met Leu Asp Pro Ala Glu Lys Asp Glu Lys Gly Met Pro
           115                 120                 125
   Val Thr Ala Arg Val Val Phe Val Phe Gly Pro Asp Lys Lys Leu Lys
       130                 135                 140
   Leu Ser Ile Leu Tyr Pro Ala Thr Thr Gly Arg Asn Phe Asp Glu Ile
   145                 150                 155                 160
   Leu Arg Val Val Ile Ser Leu Gln Leu Thr Ala Glu Lys Arg Val Ala
                   165                 170                 175
   Thr Pro Val Asp Trp Lys Asp Gly Asp Ser Val Met Val Leu Pro Thr
               180                 185                 190
   Ile Pro Glu Glu Glu Ala Lys Lys Leu Phe Pro Lys Gly Val Phe Thr
           195                 200                 205
   Lys Glu Leu Pro Ser Gly Lys Lys Tyr Leu Arg Tyr Thr Pro Gln Pro
       210                 215                 220
<210> 17
   <211> 331
   <212> PRT
   <213> Homo sapiens
<400> 17
   Met Ala Arg Gly Gly Arg Gly Arg Arg Leu Gly Leu Ala Leu Gly Leu
   1               5                   10                  15
   Leu Leu Ala Leu Val Leu Ala Pro Arg Val Leu Arg Ala Lys Pro Thr
               20                  25                  30
   Val Arg Lys Glu Arg Val Val Arg Pro Asp Ser Glu Leu Gly Glu Arg
           35                  40                  45
   Pro Pro Glu Asp Asn Gln Ser Phe Gln Tyr Asp His Glu Ala Phe Leu
       50                  55                  60
   Gly Lys Glu Asp Ser Lys Thr Phe Asp Gln Leu Thr Pro Asp Glu Ser
   65                  70                  75                  80
   Lys Glu Arg Leu Gly Lys Ile Val Asp Arg Ile Asp Asn Asp Gly Asp
                   85                  90                  95
   Gly Phe Val Thr Thr Glu Glu Leu Lys Thr Trp Ile Lys Arg Val Gln
               100                 105                 110
   Lys Arg Tyr Ile Phe Asp Asn Val Ala Lys Val Trp Lys Asp Tyr Asp
           115                 120                 125
   Arg Asp Lys Asp Asp Lys Ile Ser Trp Glu Glu Tyr Lys Gln Ala Thr
       130                 135                 140
   Tyr Gly Tyr Tyr Leu Gly Asn Pro Ala Glu Phe His Asp Ser Ser Asp
   145                 150                 155                 160
   His His Thr Phe Lys Lys Met Leu Pro Arg Asp Glu Arg Arg Phe Lys
                   165                 170                 175
   Ala Ala Asp Leu Asn Gly Asp Leu Thr Ala Thr Arg Glu Glu Phe Thr
               180                 185                 190
   Ala Phe Leu His Pro Glu Glu Phe Glu His Met Lys Glu Ile Val Val
           195                 200                 205
   Leu Glu Thr Leu Glu Asp Ile Asp Lys Asn Gly Asp Gly Phe Val Asp
       210                 215                 220
   Gln Asp Glu Tyr Ile Ala Asp Met Phe Ser His Glu Glu Asn Gly Pro
   225                 230                 235                 240
   Glu Pro Asp Trp Val Leu Ser Glu Arg Glu Gln Phe Asn Glu Phe Arg
                   245                 250                 255
   Asp Leu Asn Lys Asp Gly Lys Leu Asp Lys Asp Glu Ile Arg His Trp
               260                 265                 270
   Ile Leu Pro Gln Asp Tyr Asp His Ala Gln Ala Glu Ala Arg His Leu
           275                 280                 285
   Val Tyr Glu Ser Asp Lys Asn Lys Asp Glu Lys Leu Thr Lys Glu Glu
       290                 295                 300
   Ile Leu Glu Asn Trp Asn Met Phe Val Gly Ser Gln Ala Thr Asn Tyr
   305                 310                 315                 320
   Gly Glu Asp Leu Thr Lys Asn His Asp Glu Leu
                   325                 330
<210> 18
   <211> 654
   <212> PRT
   <213> Homo sapiens
<400> 18
   Met Lys Leu Ser Leu Val Ala Ala Met Leu Leu Leu Leu Ser Ala Ala
   1               5                   10                  15
   Arg Ala Glu Glu Glu Asp Lys Lys Glu Asp Val Gly Thr Val Val Gly
               20                  25                  30
   Ile Asp Leu Gly Thr Thr Tyr Ser Cys Val Gly Val Phe Lys Asn Gly
           35                  40                  45
   Arg Val Glu Ile Ile Ala Asn Asp Gln Gly Asn Arg Ile Thr Pro Ser
       50                  55                  60
   Tyr Val Ala Phe Thr Pro Glu Gly Glu Arg Leu Ile Gly Asp Ala Ala
   65                  70                  75                  80
   Lys Asn Gln Leu Thr Ser Asn Pro Glu Asn Thr Val Phe Asp Ala Lys
                   85                  90                  95
   Arg Leu Ile Gly Arg Thr Trp Asn Asp Pro Ser Val Gln Gln Asp Ile
               100                 105                 110
   Lys Phe Leu Pro Phe Lys Val Val Glu Lys Lys Thr Lys Pro Tyr Ile
           115                 120                 125
   Gln Val Asp Ile Gly Gly Gly Gln Thr Lys Thr Phe Ala Pro Glu Glu
       130                 135                 140
   Ile Ser Ala Met Val Leu Thr Lys Met Lys Glu Thr Ala Glu Ala Tyr
   145                 150                 155                 160
   Leu Gly Lys Lys Val Thr His Ala Val Val Thr Val Pro Ala Tyr Phe
                   165                 170                 175
   Asn Asp Ala Gln Arg Gln Ala Thr Lys Asp Ala Gly Thr Ile Ala Gly
               180                 185                 190
   Leu Asn Val Met Arg Ile Ile Asn Glu Pro Thr Ala Ala Ala Ile Ala
           195                 200                 205
   Tyr Gly Leu Asp Lys Arg Glu Gly Glu Lys Asn Ile Leu Val Phe Asp
       210                 215                 220
   Leu Gly Gly Gly Thr Phe Asp Val Ser Leu Leu Thr Ile Asp Asn Gly
   225                 230                 235                 240
   Val Phe Glu Val Val Ala Thr Asn Gly Asp Thr His Leu Gly Gly Glu
                   245                 250                 255
   Asp Phe Asp Gln Arg Val Met Glu His Phe Ile Lys Leu Tyr Lys Lys
               260                 265                 270
   Lys Thr Gly Lys Asp Val Arg Lys Asp Asn Arg Ala Val Gln Lys Leu
           275                 280                 285
   Arg Arg Glu Val Glu Lys Ala Lys Arg Ala Leu Ser Ser Gln His Gln
       290                 295                 300
   Ala Arg Ile Glu Ile Glu Ser Phe Tyr Glu Gly Glu Asp Phe Ser Glu
   305                 310                 315                 320
   Thr Leu Thr Arg Ala Lys Phe Glu Glu Leu Asn Met Asp Leu Phe Arg
                   325                 330                 335
   Ser Thr Met Lys Pro Val Gln Lys Val Leu Glu Asp Ser Asp Leu Lys
               340                 345                 350
   Lys Ser Asp Ile Asp Glu Ile Val Leu Val Gly Gly Ser Thr Arg Ile
           355                 360                 365
   Pro Lys Ile Gln Gln Leu Val Lys Glu Phe Phe Asn Gly Lys Glu Pro
       370                 375                 380
   Ser Arg Gly Ile Asn Pro Asp Glu Ala Val Ala Tyr Gly Ala Ala Val
   385                 390                 395                 400
   Gln Ala Gly Val Leu Ser Gly Asp Gln Asp Thr Gly Asp Leu Val Leu
                   405                 410                 415
   Leu Asp Val Cys Pro Leu Thr Leu Gly Ile Glu Thr Val Gly Gly Val
               420                 425                 430
   Met Thr Lys Leu Ile Pro Arg Asn Thr Val Val Pro Thr Lys Lys Ser
           435                 440                 445
   Gln Ile Phe Ser Thr Ala Ser Asp Asn Gln Pro Thr Val Thr Ile Lys
       450                 455                 460
   Val Tyr Glu Gly Glu Arg Pro Leu Thr Lys Asp Asn His Leu Leu Gly
   465                 470                 475                 480
   Thr Phe Asp Leu Thr Gly Ile Pro Pro Ala Pro Arg Gly Val Pro Gln
                   485                 490                 495
   Ile Glu Val Thr Phe Glu Ile Asp Val Asn Gly Ile Leu Arg Val Thr
               500                 505                 510
   Ala Glu Asp Lys Gly Thr Gly Asn Lys Asn Lys Ile Thr Ile Thr Asn
           515                 520                 525
   Asp Gln Asn Arg Leu Thr Pro Glu Glu Ile Glu Arg Met Val Asn Asp
       530                 535                 540
   Ala Glu Lys Phe Ala Glu Glu Asp Lys Lys Leu Lys Glu Arg Ile Asp
   545                 550                 555                 560
   Thr Arg Asn Glu Leu Glu Ser Tyr Ala Tyr Ser Leu Lys Asn Gln Ile
                   565                 570                 575
   Gly Asp Lys Glu Lys Leu Gly Gly Lys Leu Ser Ser Glu Asp Lys Glu
               580                 585                 590
   Thr Met Glu Lys Ala Val Glu Glu Lys Ile Glu Trp Leu Glu Ser His
           595                 600                 605
   Gln Asp Ala Asp Ile Glu Asp Phe Lys Ala Lys Lys Lys Glu Leu Glu
       610                 615                 620
   Glu Ile Val Gln Pro Ile Ile Ser Lys Leu Tyr Gly Ser Ala Gly Pro
   625                 630                 635                 640
   Pro Pro Thr Gly Glu Glu Asp Thr Ala Glu Lys Asp Glu Leu
                   645                 650
<210> 19
   <211> 466
   <212> PRT
   <213> Homo sapiens
<400> 19
   Met Ser Thr Arg Ser Val Ser Ser Ser Ser Tyr Arg Arg Met Phe Gly
   1               5                   10                  15
   Gly Pro Gly Thr Ala Ser Arg Pro Ser Ser Ser Arg Ser Tyr Val Thr
               20                  25                  30
   Thr Ser Thr Arg Thr Tyr Ser Leu Gly Ser Ala Leu Arg Pro Ser Thr
           35                  40                  45
   Ser Arg Ser Leu Tyr Ala Ser Ser Pro Gly Gly Val Tyr Ala Thr Arg
       50                  55                  60
   Ser Ser Ala Val Arg Leu Arg Ser Ser Val Pro Gly Val Arg Leu Leu
   65                  70                  75                  80
   Gln Asp Ser Val Asp Phe Ser Leu Ala Asp Ala Ile Asn Thr Glu Phe
                   85                  90                  95
   Lys Asn Thr Arg Thr Asn Glu Lys Val Glu Leu Gln Glu Leu Asn Asp
               100                 105                 110
   Arg Phe Ala Asn Tyr Ile Asp Lys Val Arg Phe Leu Glu Gln Gln Asn
           115                 120                 125
   Lys Ile Leu Leu Ala Glu Leu Glu Gln Leu Lys Gly Gln Gly Lys Ser
       130                 135                 140
   Arg Leu Gly Asp Leu Tyr Glu Glu Glu Met Arg Glu Leu Arg Arg Gln
   145                 150                 155                 160
   Val Asp Gln Leu Thr Asn Asp Lys Ala Arg Val Glu Val Glu Arg Asp
                   165                 170                 175
   Asn Leu Ala Glu Asp Ile Met Arg Leu Arg Glu Lys Leu Gln Glu Glu
               180                 185                 190
   Met Leu Gln Arg Glu Glu Ala Glu Asn Thr Leu Gln Ser Phe Arg Gln
           195                 200                 205
   Asp Val Asp Asn Ala Ser Leu Ala Arg Leu Asp Leu Glu Arg Lys Val
       210                 215                 220
   Glu Ser Leu Gln Glu Glu Ile Ala Phe Leu Lys Lys Leu His Glu Glu
   225                 230                 235                 240
   Glu Ile Gln Glu Leu Gln Ala Gln Ile Gln Glu Gln His Val Gln Ile
                   245                 250                 255
   Asp Val Asp Val Ser Lys Pro Asp Leu Thr Ala Ala Leu Arg Asp Val
               260                 265                 270
   Arg Gln Gln Tyr Glu Ser Val Ala Ala Lys Asn Leu Gln Glu Ala Glu
           275                 280                 285
   Glu Trp Tyr Lys Ser Lys Phe Ala Asp Leu Ser Glu Ala Ala Asn Arg
       290                 295                 300
   Asn Asn Asp Ala Leu Arg Gln Ala Lys Gln Glu Ser Thr Glu Tyr Arg
   305                 310                 315                 320
   Arg Gln Val Gln Ser Leu Thr Cys Glu Val Asp Ala Leu Lys Gly Thr
                   325                 330                 335
   Asn Glu Ser Leu Glu Arg Gln Met Arg Glu Met Glu Glu Asn Phe Ala
               340                 345                 350
   Val Glu Ala Ala Asn Tyr Gln Asp Thr Ile Gly Arg Leu Gln Asp Glu
           355                 360                 365
   Ile Gln Asn Met Lys Glu Glu Met Ala Arg His Leu Arg Glu Tyr Gln
       370                 375                 380
   Asp Leu Leu Asn Val Lys Met Ala Leu Asp Ile Glu Ile Ala Thr Tyr
   385                 390                 395                 400
   Arg Lys Leu Leu Glu Gly Glu Glu Ser Arg Ile Ser Leu Pro Leu Pro
                   405                 410                 415
   Asn Phe Ser Ser Leu Asn Leu Arg Glu Thr Asn Leu Asp Ser Leu Pro
               420                 425                 430
   Leu Val Asp Thr His Ser Lys Arg Thr Leu Leu Ile Lys Thr Val Glu
           435                 440                 445
   Thr Arg Asp Gly Gln Val Ile Asn Glu Thr Ser Gln His His Asp Asp
       450                 455                 460
   Leu Glu
   465
<210> 20
   <211> 434
   <212> PRT
   <213> Homo sapiens
<400> 20
   Met Ser Ile Leu Lys Ile His Ala Arg Glu Ile Phe Asp Ser Arg Gly
   1               5                   10                  15
   Asn Pro Thr Val Glu Val Asp Leu Phe Thr Ser Lys Gly Leu Phe Arg
               20                  25                  30
   Ala Ala Val Pro Ser Gly Ala Ser Thr Gly Ile Tyr Glu Ala Leu Glu
           35                  40                  45
   Leu Arg Asp Asn Asp Lys Thr Arg Tyr Met Gly Lys Gly Val Ser Lys
       50                  55                  60
   Ala Val Glu His Ile Asn Lys Thr Ile Ala Pro Ala Leu Val Ser Lys
   65                  70                  75                  80
   Lys Leu Asn Val Thr Glu Gln Glu Lys Ile Asp Lys Leu Met Ile Glu
                   85                  90                  95
   Met Asp Gly Thr Glu Asn Lys Ser Lys Phe Gly Ala Asn Ala Ile Leu
               100                 105                 110
   Gly Val Ser Leu Ala Val Cys Lys Ala Gly Ala Val Glu Lys Gly Val
           115                 120                 125
   Pro Leu Tyr Arg His Ile Ala Asp Leu Ala Gly Asn Ser Glu Val Ile
       130                 135                 140
   Leu Pro Val Pro Ala Phe Asn Val Ile Asn Gly Gly Ser His Ala Gly
   145                 150                 155                 160
   Asn Lys Leu Ala Met Gln Glu Phe Met Ile Leu Pro Val Gly Ala Ala
                   165                 170                 175
   Asn Phe Arg Glu Ala Met Arg Ile Gly Ala Glu Val Tyr His Asn Leu
               180                 185                 190
   Lys Asn Val Ile Lys Glu Lys Tyr Gly Lys Asp Ala Thr Asn Val Gly
           195                 200                 205
   Asp Glu Gly Gly Phe Ala Pro Asn Ile Leu Glu Asn Lys Glu Gly Leu
       210                 215                 220
   Glu Leu Leu Lys Thr Ala Ile Gly Lys Ala Gly Tyr Thr Asp Lys Val
   225                 230                 235                 240
   Val Ile Gly Met Asp Val Ala Ala Ser Glu Phe Phe Arg Ser Gly Lys
                   245                 250                 255
   Tyr Asp Leu Asp Phe Lys Ser Pro Asp Asp Pro Ser Arg Tyr Ile Ser
               260                 265                 270
   Pro Asp Gln Leu Ala Asp Leu Tyr Lys Ser Phe Ile Lys Asp Tyr Pro
           275                 280                 285
   Val Val Ser Ile Glu Asp Pro Phe Asp Gln Asp Asp Trp Gly Ala Trp
       290                 295                 300
   Gln Lys Phe Thr Ala Ser Ala Gly Ile Gln Val Val Gly Asp Asp Leu
   305                 310                 315                 320
   Thr Val Thr Asn Pro Lys Arg Ile Ala Lys Ala Val Asn Glu Lys Ser
                   325                 330                 335
   Cys Asn Cys Leu Leu Leu Lys Val Asn Gln Ile Gly Ser Val Thr Glu
               340                 345                 350
   Ser Leu Gln Ala Cys Lys Leu Ala Gln Ala Asn Gly Trp Gly Val Met
           355                 360                 365
   Val Ser His Arg Ser Gly Glu Thr Glu Asp Thr Phe Ile Ala Asp Leu
       370                 375                 380
   Val Val Gly Leu Cys Thr Gly Gln Ile Lys Thr Gly Ala Pro Cys Arg
   385                 390                 395                 400
   Ser Glu Arg Leu Ala Lys Tyr Asn Gln Leu Leu Arg Ile Glu Glu Glu
                   405                 410                 415
   Leu Gly Ser Lys Ala Lys Phe Ala Gly Arg Asn Phe Arg Asn Pro Leu
               420                 425                 430
   Ala Lys
<210> 21
   <211> 198
   <212> PRT
   <213> Homo sapiens
<400> 21
   Met Ala Ser Gly Asn Ala Arg Ile Gly Lys Pro Ala Pro Asp Phe Lys
   1               5                   10                  15
   Ala Thr Ala Val Val Asp Gly Ala Phe Lys Glu Val Lys Leu Ser Asp
               20                  25                  30
   Tyr Lys Gly Lys Tyr Val Val Leu Phe Phe Tyr Pro Leu Asp Phe Thr
           35                  40                  45
   Phe Val Cys Pro Thr Glu Ile Ile Ala Phe Ser Asn Arg Ala Glu Asp
       50                  55                  60
   Phe Arg Lys Leu Gly Cys Glu Val Leu Gly Val Ser Val Asp Ser Gln
   65                  70                  75                  80
   Phe Thr His Leu Ala Trp Ile Asn Thr Pro Arg Lys Glu Gly Gly Leu
                   85                  90                  95
   Gly Pro Leu Asn Ile Pro Leu Leu Ala Asp Val Thr Arg Arg Leu Ser
               100                 105                 110
   Glu Asp Tyr Gly Val Leu Lys Thr Asp Glu Gly Ile Ala Tyr Arg Gly
           115                 120                 125
   Leu Phe Ile Ile Asp Gly Lys Gly Val Leu Arg Gln Ile Thr Val Asn
       130                 135                 140
   Asp Leu Pro Val Gly Arg Ser Val Asp Glu Ala Leu Arg Leu Val Gln
   145                 150                 155                 160
   Ala Phe Gln Tyr Thr Asp Glu His Gly Glu Val Cys Pro Ala Gly Trp
                   165                 170                 175
   Lys Pro Gly Ser Asp Thr Ile Lys Pro Asn Val Asp Asp Ser Lys Glu
               180                 185                 190
   Tyr Phe Ser Lys His Asn
           195
<210> 22
   <211> 256
   <212> PRT
   <213> Homo sapiens
<400> 22
   Met Ala Ala Ala Val Gly Arg Leu Leu Arg Ala Ser Val Ala Arg His
   1               5                   10                  15
   Val Ser Ala Ile Pro Trp Gly Ile Ser Ala Thr Ala Ala Leu Arg Pro
               20                  25                  30
   Ala Ala Cys Gly Arg Thr Ser Leu Thr Asn Leu Leu Cys Ser Gly Ser
           35                  40                  45
   Ser Gln Ala Lys Leu Phe Ser Thr Ser Ser Ser Cys His Ala Pro Ala
       50                  55                  60
   Val Thr Gln His Ala Pro Tyr Phe Lys Gly Thr Ala Val Val Asn Gly
   65                  70                  75                  80
   Glu Phe Lys Asp Leu Ser Leu Asp Asp Phe Lys Gly Lys Tyr Leu Val
                   85                  90                  95
   Leu Phe Phe Tyr Pro Leu Asp Phe Thr Phe Val Cys Pro Thr Glu Ile
               100                 105                 110
   Val Ala Phe Ser Asp Lys Ala Asn Glu Phe His Asp Val Asn Cys Glu
           115                 120                 125
   Val Val Ala Val Ser Val Asp Ser His Phe Ser His Leu Ala Trp Ile
       130                 135                 140
   Asn Thr Pro Arg Lys Asn Gly Gly Leu Gly His Met Asn Ile Ala Leu
   145                 150                 155                 160
   Leu Ser Asp Leu Thr Lys Gln Ile Ser Arg Asp Tyr Gly Val Leu Leu
                   165                 170                 175
   Glu Gly Ser Gly Leu Ala Leu Arg Gly Leu Phe Ile Ile Asp Pro Asn
               180                 185                 190
   Gly Val Ile Lys His Leu Ser Val Asn Asp Leu Pro Val Gly Arg Ser
           195                 200                 205
   Val Glu Glu Thr Leu Arg Leu Val Lys Ala Phe Gln Tyr Val Glu Thr
       210                 215                 220
   His Gly Glu Val Cys Pro Ala Asn Trp Thr Pro Asp Ser Pro Thr Ile
   225                 230                 235                 240
   Lys Pro Ser Pro Ala Ala Ser Lys Glu Tyr Phe Gln Lys Val Asn Gln
                   245                 250                 255
<210> 23
   <211> 201
   <212> PRT
   <213> Homo sapiens
<400> 23
   Met Ala Ala Ala Lys Asp Thr His Glu Asp His Asp Thr Ser Thr Glu
   1               5                   10                  15
   Asn Thr Asp Glu Ser Asn His Asp Pro Gln Phe Glu Pro Ile Val Ser
               20                  25                  30
   Leu Pro Glu Gln Glu Ile Lys Thr Leu Glu Glu Asp Glu Glu Glu Leu
           35                  40                  45
   Phe Lys Met Arg Ala Lys Leu Phe Arg Phe Ala Ser Glu Asn Asp Leu
       50                  55                  60
   Pro Glu Trp Lys Glu Arg Gly Thr Gly Asp Val Lys Leu Leu Lys His
   65                  70                  75                  80
   Lys Glu Lys Gly Ala Ile Arg Leu Leu Met Arg Arg Asp Lys Thr Leu
                   85                  90                  95
   Lys Ile Cys Ala Asn His Tyr Ile Thr Pro Met Met Glu Leu Lys Pro
               100                 105                 110
   Asn Ala Gly Ser Asp Arg Ala Trp Val Trp Asn Thr His Ala Asp Phe
           115                 120                 125
   Ala Asp Glu Cys Pro Lys Pro Glu Leu Leu Ala Ile Arg Phe Leu Asn
       130                 135                 140
   Ala Glu Asn Ala Gln Lys Phe Lys Thr Lys Phe Glu Glu Cys Arg Lys
   145                 150                 155                 160
   Glu Ile Glu Glu Arg Glu Lys Lys Ala Gly Ser Gly Lys Asn Asp His
                   165                 170                 175
   Ala Glu Lys Val Ala Glu Lys Leu Glu Ala Leu Ser Val Lys Glu Glu
               180                 185                 190
   Thr Lys Glu Asp Ala Glu Glu Lys Gln
           195                 200
<210> 24
   <211> 215
   <212> PRT
   <213> Homo sapiens
<400> 24
   Met Gly Lys Gly Asp Pro Lys Lys Pro Arg Gly Lys Met Ser Ser Tyr
   1               5                   10                  15
   Ala Phe Phe Val Gln Thr Cys Arg Glu Glu His Lys Lys Lys His Pro
               20                  25                  30
   Asp Ala Ser Val Asn Phe Ser Glu Phe Ser Lys Lys Cys Ser Glu Arg
           35                  40                  45
   Trp Lys Thr Met Ser Ala Lys Glu Lys Gly Lys Phe Glu Asp Met Ala
       50                  55                  60
   Lys Ala Asp Lys Ala Arg Tyr Glu Arg Glu Met Lys Thr Tyr Ile Pro
   65                  70                  75                  80
   Pro Lys Gly Glu Thr Lys Lys Lys Phe Lys Asp Pro Asn Ala Pro Lys
                   85                  90                  95
   Arg Pro Pro Ser Ala Phe Phe Leu Phe Cys Ser Glu Tyr Arg Pro Lys
               100                 105                 110
   Ile Lys Gly Glu His Pro Gly Leu Ser Ile Gly Asp Val Ala Lys Lys
           115                 120                 125
   Leu Gly Glu Met Trp Asn Asn Thr Ala Ala Asp Asp Lys Gln Pro Tyr
       130                 135                 140
   Glu Lys Lys Ala Ala Lys Leu Lys Glu Lys Tyr Glu Lys Asp Ile Ala
   145                 150                 155                 160
   Ala Tyr Arg Ala Lys Gly Lys Pro Asp Ala Ala Lys Lys Gly Val Val
                   165                 170                 175
   Lys Ala Glu Lys Ser Lys Lys Lys Lys Glu Glu Glu Glu Asp Glu Glu
               180                 185                 190
   Asp Glu Glu Asp Glu Glu Glu Glu Glu Asp Glu Glu Asp Glu Asp Glu
           195                 200                 205
   Glu Glu Asp Asp Asp Asp Glu
       210                 215